# NEUE EUROPÄISCHE PATENTSCHRIFT

(11) **EP 1 725 201 B2**
(45) Veröffentlichungstag und Bekanntmachung des Hinweises auf die Entscheidung über den Einspruch: **11.09.2013**
(45) Hinweis auf die Patenterteilung: 31.12.2008
(21) Anmeldenummer: 05706749.8
(22) Anmeldetag: 08.02.2005
(51) Int. Cl.: A61F 13/58

(54) **VERSCHLUSSBAND FÜR EINEN HYGIENEARTIKEL, WINDEL, VERFAHREN ZUM SCHLIESSEN EINER WINDEL, BANDMATERIAL UND WICKLUNG EINES BANDMATERIALS**
CLOSING STRIP FOR A HYGIENE ARTICLE, DIAPER, METHOD FOR CLOSING A DIAPER, STRIP MATERIAL AND WINDING OF A STRIP MATERIAL
RUBAN DE FERMETURE POUR ARTICLE HYGIENIQUE, COUCHE, PROCEDE DE FERMETURE D'UNE COUCHE, MATERIAU SOUS FORME DE RUBAN ET BOBINE DE MATERIAU SOUS FORME DE RUBAN

(30) Priorität: 08.02.2004 DE 102004006219; 08.02.2004 DE 102004006221
(43) Veröffentlichungstag der Anmeldung: 29.11.2006
(73) Patentinhaber: Koester GmbH & Co. KG, 96146 Altendorf (DE)
(72) Erfinder: NEUGEBAUER, Robert, 91077 Neunkirchen (DE); SILBERLING, Wolfgang, 90482 Nürnberg (DE)
(74) Vertreter: Reuther, Martin
(86) Internationale Anmeldenummer: PCT/DE2005/000209
(87) Internationale Veröffentlichungsnummer: WO 2005/074852

(56) Entgegenhaltungen:
- EP-A1- 0 755 665
- EP-A1- 0 941 730
- EP-A1- 1 635 752
- EP-A1- 1 635 753
- EP-B1- 0 321 232
- EP-B1- 0 820 264
- WO-A-97/36566
- WO-A-03/003962
- WO-A1-97//36566
- WO-A1-03//003962
- GB-A- 2 296 423
- US-A1- 2002 095 130
- US-A1- 2003 004 490
- US-A1- 2003 060 794
- US-B2- 7 037 457

## Beschreibung

Die Erfindung betrifft ein Verschlussband für einen Hygieneartikel, eine Windel, ein Verfahren zum Schließen, Anlegen und/oder Entsorgen einer Windel, ein Bandmaterial und eine Wicklung eines Bandmaterials. Genauer betrifft die Erfindung solche Verschlussbänder und deren Kontext, bei welchen ein Schließbereich am Verschlussband zum lösbaren Verschließen des Hygieneartikels eine Komponente eines mechanischen ZweiKomponenten-Verschlusssystems und eine offene Kleberschicht aufweist.

Die Windelverschlussbänder werden bei der Herstellung einer Windel mit dieser verbunden, sodass ein Benutzer der Windel diese mit Hilfe der Windelverschlussbänder nach dem Anlegen verschließen kann. Oft werden hierzu zwei Windelverschlussbänder an den seitlichen Windelohren der Vorderseite oder der Rückenseite einer Windel permanent fixiert. Diese Fixierung ist so eingestellt, dass sie zumindest über die voraussehbare Lebensdauer der Windel andauert, und sie ist im Allgemeinen nicht ohne Zerstörung der Windel und/oder des Verschlussbands lösbar.

Derartige Verschlussbänder sind beispielsweise aus der EP 0 321 232 B1 bekannt. Die dortigen Windelverschlussbänder bestehen aus einem Fasteningträger, der einen Befestigungsbereich zum permanenten Befestigen an der Windel aufweist und zu einem freien, von der Windel abstehenden Benutzerende hin einen Schließbereich mit einem Hakenbereich und einer offenen Kleberschicht trägt. Der Hakenbereich und die offene Kleberfläche bilden in ihrer Gesamtfläche einen Schließbereich zum lösbaren Verschließen der Windel. Dabei kann der Hakenbereich nach einer vorgestellten Variante nicht nur am freien Ende des Schließbereichs angeordnet sein, sondern auch mittig im Schließbereich, so dass sich auf beiden Seiten des Hakenbereichs jeweils eine Teilfläche mit offenem Klebstoff bildet.

Auch die JP 8-2365 B zeigt eine Windel mit einem Windelverschlussband, an dessen Schließbereich eine oder zwei zentrale Hakenflächen sowie diese umgebende Klebestreifen vorgesehen sind.

Die FR 2 586 558 A1 zeigt eine Windel mit einem gürtelförmigen Windelverschlussband. In der Frontpartie des Backsheets können optional kleine Halteflächen mit Haken vorgesehen werden. Für diesen Fall wird vorgeschlagen, dass die Windelverschlussbänder an ihrem Schließbereich optional ein Schlaufenmaterial erhalten, während zum freien Benutzerende hin eine Klebstofffläche vorgesehen ist.

Die WO 00/27236 zeigt Windelverschlussbänder mit Hakenflächen und offenen Kleberflächen im Schließbereich in unterschiedlichsten Ausgestaltungen.

Weitere Verschlussbänder, meist für Babywindeln oder Inkontinenzwindeln, zeigen unter anderem die US 5,624,429, die EP 0 795 307 A2, die EP 0 793 953 A2, die EP 0 840 585 B1, die DE 101 29 180 A1, die EP 0 235 014 B1, die EP 0 233 704 A2, die FR 2 606 257, die US 6,210,389 B1, die US 5,759,317, die US 5,399,219, die EP 1 000 598 A1, die DE 197 32 499 A1, die WO 02/49568 A1, die WO 02/49567 A1, die WO 01/74283 A1, die WO 00/27330 A1, die EP 1 024 774 B1, die US 4,670,012, die DE 101 40 622 A1, die DE 28 56 869 A1, die DE 101 40 621 A1, die WO 97/32555 A1, die WO 96/31181 A1 die WO 97/23186 A1, die US 5,759,317, die US 5,399,219, die US 6,210,389 B1, die US 5,624,429, die EP 0 233 704 B1, die EP 0 800 378 B1, die US 6,454,751 B1, die US 5,997,522, die US 2003/0009144 A1, die US 2003/0023322, die US 2003/0034583 A1, die WO 01/67911 A2 und die WO 01/68019 A1.

Hauptaufgabe der Windelverschlussbänder ist es, die Windel sicher verschlossen zu halten und dabei möglicht keine Einbußen beim Tragekomfort zu provozieren. Sowohl bei Säuglingswindeln als auch bei Erwachsenenwindeln können unterschiedlichste Kräfte auf das Verschlusssystem der Windel einwirken.

Der hier vorliegenden Erfindung liegt die Aufgabe zugrunde, Windeln, Windelverschlussbänder und Bandmaterial zu deren Herstellung vorzustellen, die bezüglich ihres Verschlusssystems verbessert sind und/oder ein verbessertes Preis-Leistungs-Verhältnis aufweisen. Die Aufgabe wird durch ein Windelverschlussband, durch eine Windel, durch ein Verfahren zum Schließen, Anlegen und/oder Entsorgen einer Windel, durch ein Bandmaterial bzw. durch eine Wicklung eines Bandmaterials mit den Merkmalen der nebengeordneten Ansprüche gelöst.

Bei einem Verschlussband für einen Hygieneartikel, insbesondere für eine Babywindel oder für eine Inkontinenzwindel, mit einem Befestigungsbereich zum permanenten Befestigen am Hygieneartikel und mit einem Schließbereich zum gleichzeitigen lösbaren Verbinden mit einer Oberfläche des Hygieneartikels können der Schließbereich zwei Flächenbereiche aufweisen, die Komponenten unterschiedlicher Verschlusssysteme tragen und der eine Flächenbereich eine shear-off-Sicherung und der andere Flächenbereich eine pop-off-Sicherung tragen.

Hierzu sei folgendes erläutert: Verschlussbänder der hier maßgebenden Art haben einen Befestigungsbereich und einen Schließbereich. Der Befestigungsbereich hat in der Regel eine offene Haftfläche oder ähnliches, über die er permanent an beispielsweise eine Windel angeklebt werden kann. Als freies Benutzerende des Verschlussbands ist dann derjenige Teil des Bandes zu bezeichnen, welcher nicht permanent mit der Windel verklebt bzw. verbunden ist. Das Benutzerende des Verschlussbandes dient dazu, dass der Benutzer es zum Verschließen der Windel nach dem Anlegen oder nach einem Zusammenfalten nach dem Ablegen ergreifen und auf eine hierfür vorgesehene Landezone an einer Frontpartie des Backsheets der Windel führen kann. Zum Benutzerende hin weist das Verschlussband einen Schließbereich auf, der beispielsweise sowohl eine mechanische Verschlusskomponente, insbesondere einen Hakenbereich, und eine Kleberfläche aufweisen kann, wobei dann die shear-off-Sicherung durch die mechanische Verschlusskomponente und die pop-off-Sicherung durch die Kleberfläche gebildet werden. Der Benutzer drückt zum Verschließen der Windel diesen Doppelfunktion-Schließbereich gegen die Landezone. Dies führt zu einem Schluss des mechanischen Zwei-Komponenten-Verschlusssystems, gleichzeitig verklebt die offene Kleberfläche des Schließbereichs lösbar mit der Landezone.

Eine solche Gattung von Windelverschlussband kann beispielsweise der EP 0 321 232 B1 und der JP 8-2365 B entnommen werden. Die mechanische Verschlusskomponente wechselwirkt mit einem Frontaltape, welches auf dem Backsheet der Windel an der Frontpartie angeordnet ist und die komplementäre Komponente des mechanischen Verschlusssystems trägt. Die Klebstoffschicht wechselwirkt mit entsprechenden Folienbereichen auf dem Backsheet, die sich zum Auflegen des Verschlussbands anbieten, wenn die Windel zum Entsorgen zusammengefaltet ist - oder sie wechselwirkt mit Folienbereichen auf einem Releasetape, welches dergestalt angeordnet ist, dass es sich zum lösbaren Befestigen des Schließbereichs während der Fertigung der Windeln anbietet. Insofern wird nach dem Stand der Technik eine Aufgabenverteilung vorgenommen, gemäß welcher das Verschlussband während des Tragens der Windel über die mechanische Verschlusskomponente geschlossen gehalten werden soll, während bei der Fertigung und zum Entsorgen diese Aufgabe einer Verbindung zwischen der Klebeschicht des Schließbereichs und einer hierfür vorgesehenen Aufnahmefolie zukommt.

Die Erfindung erkennt demgegenüber, dass ein Schließbereich mit zwei verschiedenen Sicherungen, die jeweils in ihren Aufgaben spezialisiert sind, nämlich einer pop-off-Sicherung, wie beispielsweise einer Klebeschicht, und einer shear-off-Sicherung, wie beispielsweise einer mechanischen Komponente, mit diesen vereinten Kräften hervorragend zum Geschlossenhalten der Windel sowohl bei der Fertigung als auch während des Tragens als auch zum Entsorgen genutzt werden kann.

Dementsprechend muss erfindungsgemäß die shear-off-Sicherung, also die in dem ersten Flächenbereich angeordnete Komponente eines ersten Verschlusssystems, senkrecht auf das Band wirkenden Kräften stärker entgegenwirken als dieses durch die in dem zweiten Flächenbereich angeordnete Komponente des zweiten Verschlusssystems der Fall ist. Umgekehrt muss erfindungsgemäß die pop-off-Sicherung, also die in dem zweiten Flächenbereich angeordnete Komponente des zweiten Verschlusssystems, etwaigen Kräften, die parallel auf das Band wirken, stärker entgegenwirken als dieses durch die in dem ersten Flächenbereich angeordnete Komponente des ersten Verschlusssystems der Fall ist. Besonders bevorzugt sind diese Kraftverhaltnisse auch bei den Kräften je Flächeneinheit entsprechend zu finden, was jedoch nicht zwingend der Fall sein muss.

Die pop-off-Sicherung sorgt in starkem Maße dafür, dass das Verschlussband nicht in einer Richtung senkrecht zur Bandfläche von seiner Landezone abspringen kann oder abgezogen werden kann. Die pop-off-Sicherung ist somit bevorzugt eine Klebstoffschicht, die mit einem textilen Material, insbesondere dem Backsheet, wechselwirken kann.

Die shear-off-Sicherung dient im starken Maße dazu, ein Aufeinander-Abgleiten des Verschlussbands über die Landezone zu verhindern. Es erschwert somit ein Abscheren zwischen Verschlussband und Landezone in der Ebene des Verschlussbands. Es nimmt insbesondere die Scherkräfte zwischen Landezone und Verschlussband auf. Hierzu ist beispielsweise eine mechanische Haken-Schlaufen-Verbindung sehr geeignet.

Es versteht sich, dass die vorstehend beschriebene Verteilung der Aufgabenschwerpunkte zwischen pop-off-Sicherung und shear-off-Sicherung nicht ausschließlich ist. Insbesondere wird das mechanische Verschlusssystem auch einen Beitrag zum Zusammenhalten des Verschlusses normal zur Ebene des Verschlussbands liefern. Ebenso wird die Klebstoffverbindung einen Teil der Scherkräfte aufnehmen. Es hat sich jedoch gezeigt, dass im Zusammenspiel aus mechanischer Verbindung und Klebeverbindung das mechanische Verschlusssystem insbesondere den Scherkräften entgegentritt, während der Klebstoff ein senkrechtes Abheben des Bands von dem textilen Material verhindert, so dass der mechanische Verschluss weiter wirksam bleiben kann.

Nach einem bevorzugten Aspekt der Erfindung ist zwischen der shear-off Sicherung und dem freien Ende des Verschlussbands der verbleibende Schließbereich flächenmäßig größer ist als die shear-off-Sicherung.

Konsequenterweise muss dieser Aspekt der Erfindung eine ausreichende Kraft zum sicheren Geschlossenhalten der Windel unter Zuhilfenahme beider Verschlussarten gewährleisten. Unter dem als Aufgabe präsenten Gesichtspunkt des verbesserten Kosten-Nutzen-Verhältnisses und der Verschlussqualität schlägt der vorgestellte Aspekt der Erfindung daher diesen Aufbau vor: Vom freien Benutzerende aus soll der Schließbereich mit einer pop-off-Sicherung beginnen. Auf gleicher Höhe des Verschlussbands - dann dementsprechend nur über einen Teil der Bandbreite - kann auch schon eine shear-off-Sicherung vorliegen. Maßgebend ist jedoch, dass zumindest ein Teil der zum freien Benutzerende hin liegenden Begrenzungskante des Schließbereichs eine pop-off-Sicherung aufweist.

Angesichts der schwerpunktmäßigen Aufgabenverteilung ist es von Vorteil, dass am freien Benutzerende zunächst die pop-off-Sicherung vorgesehen ist. Scherkräfte zwischen dem Band und der Landezone sollen bereits im näher zum Befestigungsbereich liegenden Teil des Schließbereichs aufgenommen werden. Am zum freien Benutzerende hinweisenden Ende des Schließbereichs dürften somit keine oder nur noch äußerst geringe Scherkräfte vorliegen. Insofern besteht die notwendige Aufgabe an diesem Ende zumindest weitgehend darin, ein ungewolltes Abfallen des Verschlussbandes senkrecht zur Bandebene zu verhindern.

Bei einer solchen Konstellation wird nach dem vorstehend genannten Aspekt also vorgeschlagen, dass der verbleibende Schließbereich zwischen der shear-off-Sicherung und dem freien Ende größer ist als die shear-off-Sicherung. Hierdurch wird der Gedanke der Aufgabenverteilung konsequent zu Ende gedacht und kostengünstig angewendet. Eine Fläche mit einer mechanischen Verschlusskomponente zu bedecken, beispielsweise mit Haken, ist deutlich aufwändiger und kostenintensiver als dieselbe Fläche mit einer Klebeschicht zu versehen. Die Erfindung hat erkannt, dass bereits eine recht kleine Hakenfläche im Schließbereich ausreicht, um die beim Tragen und beim Entsorgen einer Windel auftretenden üblichen Scherkräfte aufzunehmen. Die Hakenfläche kann somit recht klein gehalten werden und insbesondere die Hälfte der Gesamtfläche des Schließbereichs unterschreiten. Auf diese Weise wird eine vergrößerte Fläche für die - im Allgemeinen recht preiswert ausführbare - pop-off-Sicherung nutzbar. Insbesondere kann sogar die Fläche der pop-off-Sicherung größer als die Fläche der shear-off-Sicherung sein. Die große Klebefläche nach dem vorgestellten Aspekt der Erfindung korrespondiert also mit der relativ kleinen Fläche der mechanischen Verbindungskomponente im Schließbereich des Verschlussbands.

Gleichzeitig ermöglicht es die vorgeschlagene Anordnung, dass das mechanische Verschlusssystem nicht ganz so leistungsfähig ausgestaltet sein muss. Insbesondere dann, wenn der verbleibende Schließbereich zwischen der shear-off-Sicherung und dem freien Ende nicht allzu viel größer ist als die shear-off-Sicherung, kann beispielsweise bei einem Haken-Schlaufen-Verschlusssystem das textile Material, welches die Schlaufen bereitstellt, minderwertiger gewählt werden. Insofern können beispielsweise an die textile Struktur eines Frontaltapes, welches die Landezone für das Verschlussband verkörpert und nach dem Stand der Technik große Maschen aufweisen muss, niedrigere Anforderungen gestellt werden. Dabei können beispielsweise auch einfache Nonwoven-Materialien zur Anwendung kommen.

Dies ermöglicht es insbesondere, dass auf ein Frontaltape unter Umständen vollständig verzichtet werden kann. In diesem Fall wird das erfindungsaspektgemäße Windelverschlussband unmittelbar auf das Nonwoven auf dem Backsheet der Windel gedrückt. Das Frontaltape kann entfallen. Hierdurch lassen sich die Gesamtkosten für eine Windel erheblich reduzieren.

Im vorliegenden Zusammenhang beschreibt der Begriff "Haken-Schlaufen-Befestigungssystem" bzw. mechanisches Verschlusssystem ein Befestigungssystem, bei welchem aus einer Oberfläche hervorstehende Anordnung einer ersten, männlichen Komponente hinter Aussparungen, Schlaufen, Öffnungen, Fasern oder ähnliches einer zweiten, weiblichen Komponente greifen können und somit einen Widerstand gegen ein Verscheren und/oder ein Ablösen hervorrufen. Anders als adhäsive Verschlusssysteme, bei welchen durch Adhäsion, beispielsweise durch Klebstoff, eine entsprechende Verbindung zweier Baugruppen gewährleistet wird.

Zur genauen Anordnung von pop-off-Sicherung und shear-off-Sicherung im Schließbereich wird vorgeschlagen, dass die shear-off-Sicherung eher zum Befestigungsbereich des Verschlussbands hin verlagert wird. Eine pop-off-Sicherung auf der zum Befestigungsbereich hinweisenden Seite der shear-off-Sicherung ist im Vergleich zu einer Anordnung ihrer gleichen Fläche wie vorgeschlagen deutlich unwirksamer. Insofern wird sogar vorgeschlagen, dass die pop-off-Sicherung auf der zum freien Benutzerende hin liegenden Seite der gesamten shear-off-Sicherung größer ist als die Fläche dieser gesamten shear-off-Sicherung.

Es versteht sich, dass als Klebstoff zumindest im Schließbereich ein drucksensitiver Klebstoff eingesetzt werden sollte bzw. eingesetzt werden kann.

Da es nach dem bereits vorgestellten Aspekt der Erfindung möglich ist, auf ein Frontaltape zu verzichten, schlägt ein weiterer Aspekt der Erfindung ein Verfahren zum Schließen einer Windel zum Anlegen und/oder zum Entsorgen vor, wobei zum Schließen eine Verbindung zwischen einem Schließbereich eines Windelverschlussbands an einem Windelohr und einer Landezone, vor allem an einer Frontpartie, hergestellt wird, wobei sich das Verfahren dadurch auszeichnet, dass als Landezone unmittelbar ein Oberflächenbereich eines textilen Backsheets der Windel gewählt wird.

Analog zu diesem Gedanken regt ein dritter Aspekt der Erfindung eine solche Abstimmung zwischen dem Schließbereich und einem textilen Backsheet an, dass das Windelverschlussband über den Schließbereich mit einer Landezone auf dem textilen Backsheet lösbar verbindbar ist. Es ist offensichtlich, dass ein Frontaltape bei einer solchen Einstellung der kombinierten Haltekraft des Schließbereichs am Backsheet überflüssig ist. Zudem ergibt sich bei einem Verzicht auf das Frontaltape die Möglichkeit, eine riesige Landezone zu nutzen, da praktisch die gesamte Frontpartie des Backsheets hierzu verwendet werden kann. Dies ist insbesondere beim Anlegen der Windel zum Tragen, aber auch beim Entsorgen der Windel, von besonderem Vorteil. Insbesondere müssen die Windelverschlussbänder nicht mehr so stark elastisch ausgeprägt sein, wie dies bislang gefordert ist, da eine wesentlich größere Landezone zur Verfügung gestellt wird.

In einer bevorzugten Ausführungsform weist das Backsheet an seiner Außenseite ein Non-Woven-Material auf bzw. ist aus diesem gebildet. Ein Non-Woven-Material hat in Bezug auf ein mechanisches Verschlusssystem den Nachteil, dass ein Hakenmaterial bzw. der hintergreifende Teil eines mechanischen Verschlusssystems hier nur verhältnismäßig schwer einhaken kann. Insofern wird nach dem Stand der Technik gewebtes oder gewirktes Material bevorzugt, was zwar kostspieliger ist allerdings tiefere gut zugängliche Schlaufen bereitstellen kann. Als Landefläche für einen Klebstoff ist ein Non-Woven-Material, ähnlich wie jedes andere textile Material ebenfalls nicht optimal. Hier eignen sich glatte Flächen, wie aus dem Stand der Technik als Backsheets für Windeln bekannte Folien an sich besser. Durch die vorliegende Erfindung jedoch können diese Nachteile in Vorteile gewandelt werden. So haftet ein Klebstoff besser auf einem Non-Woven-Material als auf einem Gewebe bzw. Gewirke, welches für ein mechanisches Verschlusssystem ausreichend tiefe Schlaufen aufweist. Andererseits können Haken oder ähnliche Vorsprünge in das Non-Woven-Material eindringen und eine shear-off-Sicherung bereitstellen, was in Wechselwirkung mit einer Folie nicht möglich ist. Insofern kann durch die funktionelle Trennung zwischen shear-off-Sicherung und pop-off-Sicherung ein Verschlusssystem mit einem Non-Woven-Backsheet bereitgestellt werden, was eine ausreichende Schließkraft aufweist und, da ein Non-Woven-Material im Vergleich zu einem Gewirke oder Gewebe verhältnismäßig kostengünstig ist, auf ein separates Frontaltape verzichtet werden, so dass die gesamte Windelaußenseite eine textile Haptik erhalten kann.

Alternativ bzw. kumulativ kann der Schließbereich vom Befestigungsbereich aus gesehen zunächst zumindest zum Teil die shear-off-Sicherung, vorzugsweise in Form von Haken aufweisen. Auch dies ist eine konsequente Anwendung der erfinderischen Erkenntnis, wie zwischen den beiden Systemen zum Verschließen der Windel, die im Schließbereich vorgesehen sind, wie Aufgaben und Kräfte verteilt werden können: Es wurde bereits erläutert, dass der Schließbereich zum Befestigungsbereich hin vorteilhaft zunächst eine shear-off-Sicherung aufweisen kann. Hierfür bieten sich insbesondere Haken eines Haken-Schlaufen-Verbindungssystems an. Wenn an der zum Befestigungsbereich hinweisenden Kante des Schließbereichs zunächst zumindest über einen Teil der Breite des Bands Haken vorgesehen sind, findet eine besonders gute, gezielte Kraftübertragung zwischen der Frontpartie und der Rückenpartie der Windel statt - seitlich der shear-off-Sicherung wird nur sehr wenig Kraft innerhalb der Ebene des Verschlussbands aufgenommen werden müssen. Hierfür ist die pop-off-Sicherung mit Klebstoff auch nicht primär gedacht. Insofern gibt es keine vergeudete, weil dort unpassend vorgesehene, Klebefläche. Auch dies hilft, Kosten zu sparen und einen besonders sicheren Verschluss zur Verfügung stellen.

Idealiter sollte vom Befestigungsbereich her zunächst die shear-off-Sicherung im Schließbereich vorgesehen sein, während vom Benutzerende aus zunächst die pop-off-Sicherung im Schließbereich vorgesehen ist.

Vorzugsweise umfasst das Verschlussband ein separierbares Targetband, welches am Schließbereich angeordnet ist und diesen teilweise abdeckt, zumindest einen Teil der shear-off-Sicherung aber ausspart.

Wenn das Targetband auf seiner Rückseite, also auf der vom Verschlussband abgewandten Seite, Klebstoff aufweist, kann es besonders einfach dort an der Frontpartie, genauer in der Landezone, angebracht werden, wo beim Verschließen der Windel der Schließbereich des Windelverschlussbands landen wird. In diesem Falle muss nur das Windelverschlussband gemeinsam mit dem mit Klebstoff an seiner Rückseite beschichteten Targetband ergriffen werden, die Windel in ihrem Gebrauchszustand bzw. Entsorgungszustand gebracht werden und das Windelverschlussband auf die Landezone aufgedrückt werden. Gleichzeitig mit den freigelassenen Haken des Schließbereichs trifft in diesem Falle die klebstoffbeschichtete Rückseite des Targetbands an der Landezone des Backsheets auf. Das Targetband verklebt sich augenblicklich unlösbar mit dem Backsheet und wird somit zum Frontaltape in der Landezone auf dem dortigen Backsheet. Wenn nun das Windelverschlussband von der Frontpartie abgezogen wird, verbleibt das nunmehr festgeklebte Targetband an der Frontpartie. Das ursprüngliche Windelverschlussband mit dem am Schließbereich angeordneten Targetband löst sich, so dass der Benutzer nach dem Abziehen des Verschlussbands vom Targetband lediglich das herkömmliche Windelverschlussband mit einer mechanischen Komponente und einer Kleberfläche in der Hand hält.

Wenn nach diesem Abziehen die Windel nochmals verschlossen werden soll, trifft die offene Klebefläche des Schließbereichs wieder auf das Targetband in der Landezone, während die Haken des Schließbereichs zumindest zum Teil wieder neben dem - nun als Frontaltape dienenden - Targetband an dem Backsheet der Windel in der Landezone zum Liegen kommen. Es ergibt sich somit wieder die vorteilhafte doppelte Verbindungsweise.

Wenn die Windel nach dem Benutzen entsorgt wird, ist es je nach Falttechnik wahrscheinlich, dass das Windelverschlussband an einer anderen Stelle am Backsheet der Windel zum Liegen kommt, als es beim Tragen der Windel eingenommen hatte. Auch zu diesem Zeitpunkt besteht jedoch die doppelte Verschlusswirkung des Schließbereichs: Die Haken des mechanischen Verschlusssystems verhaken sich unmittelbar mit der Oberfläche des Backsheets. Gleichzeitig hält die offene Kleberschicht des Schließbereichs das Windelverschlussband an der Oberfläche des Backsheets nieder. Durch das Niederhalten ist es nicht zwingend notwendig, dass die Haken sehr tief in das textile Material eindringen und einzelne Fasern oder Maschen beispielsweise hintergreifen, da der Klebestoff ein Herauslösen sicher verhindert.

Eine derartige Anordnung ermöglicht es insbesondere, den Klebstoff, mit welchem das Targetband auf dem Backsheet zu befestigen ist, derart zu wählen, dass er äußerst fest an diesem haftet. Dieses kann soweit gehen, dass das Targetband nicht mehr zerstörungsfrei von dem Backsheet entfernt werden kann. Letzteres ist auch nicht zwingend notwendig, da das Targetband nach dem ersten Schließen der Windel auch an seiner Position auf dem Backsheet verbleiben soll.

In einer bevorzugten Ausführungsform deckt das Targetband den Schließbereich bis auf die shear-off-Sicherung vollständig ab. Auf diese Weise wird automatisch der offenen Kleberschicht des Schließbereichs eine möglichst große perfekte Landezone zur Verfügung gestellt. Gleichzeitig sind die Haken des Schließbereichs von der Abdeckung durch das Targetband ausgenommen, wodurch dieses nur so groß wie eben nötig hergestellt werden muss.

Unabhängig von der genauen Größe des Targetbands wird vorgeschlagen, dass dieses über das Benutzerende des Windelverschlussbands hinausragt. Dieser Vorschlag wird besonders dann verständlich, wenn eine bevorzugte Form des Handlings eines Windelverschlussbands beim Anbringen an eine Windel angewendet wird: Gemäß dieser Variante weist das Windelverschlussband in seiner Ursprungsform drei unterschiedliche Lagen auf. Zunächst ist dies ein Fasteningträger. Der Fasteningträger ist ein durchlaufendes Band, welches im Wesentlichen die eigentliche Größe und Form des Windelverschlussbands definiert. Am Fasteningträger können der Befestigungsbereich und der Schließbereich ausgemacht werden. Auf dem Fasteningträger befindet sich als Befestigungsbereich eine offene Kleberschicht, die bis über den gesamten Schließbereich hinaus über den Fasteningträger verläuft. Im Schließbereich ist auf einer Teilfläche ein Hakenmaterial vorgesehen. Räumlich neben dem Hakenmaterial ist ein Targetband vorgesehen, welches die Klebstoffschicht des Schließbereichs vollständig abdeckt, aber die Haken ausspart. Auf der Rückseite des Targetbands ist eine Klebstoffschicht vorgesehen, an welcher ein antihaftbeschichtetes Releaseband angeschlossen ist. Das Releaseband weist auf seiner wiederum rückseitigen Seite auch einen Klebstoff auf. Das Releaseband endet bezüglich der Längserstreckungsrichtung des Windelverschlussbands etwa auf Höhe des Fasteningträgers. Das Targetband steht über diese beiden Enden hinaus ab, so dass ein Benutzer die dreilagige Anordnung aus Fasteningträger, Targetband und Releaseband bei einem Anfassen an der Stirnseite, also am freien Benutzerende, zunächst am Targetband ergreifen würde.

Eine Releaseband ist im Übrigen auch unabhängig von einem Targetband vorteilhaft: Das Windelverschlussband kann gemeinsam mit dem Releaseband produziert oder zumindest zur Rolle bei der Windelherstellung angeliefert werden. Das Windelverschlussband wird mit seinem Befestigungsbereich permanent an ein Windelohr der Windel angesetzt. Im Schließbereich des Windelverschlussbands ist nunmehr das Releaseband angeordnet. Es dient dazu, den Schließbereich während der Produktion, ggf. auch bis kurz vor dem Verschließen der Windel, niederzuhalten, so dass insbesondere während der Produktion der Windel kein lose abstehendes Ende vorliegt. Dies verhindert es, dass sich beispielsweise ein Windelverschlussband in der Produktionsmaschine der Windel verhaken und diese damit beschädigen kann.

Hierbei wird die Verbindung zwischen dem Releaseband und dem Schließbereich derart gewählt, dass der Schließbereich von einem Nutzer ohne weiteres ergriffen und von dem Releaseband gelöst werden kann.

Vorzugsweise bedeckt das Releaseband lediglich die pop-off-Sicherung. Hierdurch kann die Fläche für das Releaseband minimiert werden, wobei die Vorteile eines Releasebandes insbesondere im Zusammenspiel mit einer Abdeckung der pop-off-Sicherung zum Tragen kommen, da das Releaseband in seiner Oberflächenbeschaffenheit insbesondere auf die Bedürfnisse eines adhäsiven Verschlusssystems abgestellt werden kann.

Darüber hinaus ist es vorteilhaft, wenn Releaseband und/oder Targetband den shear-off-Bereich nicht überdecken, da dieser in der Regel wesentlich dicker als die Folien der Release- bzw. Targetbänder ist und auf diese Weise die Dicke des Windelverschlussbandes bzw. einer entsprechenden Bandes vergleichmäßigt werden kann. Dieses führt dazu, dass hieraus gebildete Rollen stabiler sind.

Es sei darauf hingewiesen, dass die durchlaufende Klebstoffschicht auf dem Fasteningträger nicht durchgehend aus einem Werkstoff bestehen muss. Vielmehr wird nach einem weiteren Aspekt der Erfindung vorgeschlagen, dass im Längsverlauf der Klebstofflage ein Wechsel des Klebstoffs vorgenommen wird. Beispielsweise kann der Klebstoff im Befestigungsbereich dafür vorgesehen sein, nur einmalig eine Verbindung eingehen zu müssen. Auch kann hier eine Umgebungsbedingung zum Verkleben erforderlich sein, die beim Anlegen der Windel nicht erreicht wird, beispielsweise eine sehr heiße Umgebung. Demgegenüber kann der Klebstoff im Schließbereich darauf abgestimmt sein, auch mehrfach Verbindungen mit dem Backsheet oder mit dem Targetband einnehmen und aufgeben zu können. Der Klebstoff im Schließbereich sollte drucksensitiv sein, damit er durch einfaches Andrücken durch die Bedienperson in Verbindungsschluss gebracht werden kann.

Bei einem Windelverschlussband für ein mechanisches, zwei Komponenten umfassendes Verschlusssystem, bei welchem Fasteningträger mit einem Befestigungsbereich und einem Trägerbereich eine Komponente des mechanischen Verschlusssystems in seinem Trägerbereich trägt und diese Komponente auf einem als Flecken ausgebildeten Zwischenträger angeordnet ist, welcher seinerseits an dem Fasteningträger befestigt ist und dessen Rand zumindest in einem Teilbereich schräg bezüglich einer Längserstreckungsrichtung des Windelverschlussbands angeordnet ist, kann das Windelverschlussband einen Randträger aufweisen, der als Randflecken ausgebildet ist, eine Komponente des mechanischen Verschlusssystems trägt und seinerseits innerhalb des Trägerbereichs an dem Fasteningträger befestigt ist, wobei er gleichzeitig entlang eines Randabschnitts eine Begrenzung des Trägerbereichs bildet. Hierdurch können, unabhängig von den übrigen Merkmalen vorliegender Erfindung, Windelverschlussbänder beziehungsweise Windeln zur Verfügung gestellt werden, die besonders komfortabel und zuverlässig anzulegen und zu verschließen sind und die dabei sehr preisgünstig herstellbar sind.

Es sei erläutert, dass das mechanische, zwei Komponenten umfassende Verschlusssystem insbesondere ein Haken-Schlaufen-Verschluss sein kann. Geeignet in diesem Sinne sind sämtliche zusammenwirkfähigen Oberflächen, bei welchen aus einer Oberfläche hervorstehende Anordnungen der einen Komponente hinter Aussparungen, Schlaufen, Öffnungen, Fasern oder ähnliches der anderen Komponente greifen können, sodass sich gegen ein Ablösen und/oder Verscheren der beiden Oberflächen eine Widerstandskraft ergibt. Die hervorstehenden Anordnungen der einen Komponente können insbesondere Haken oder pilzkopfförmige Stäbchen sein.

Der Befestigungsbereich des Windelverschlussbands dient dazu, selbiges permanent an einer Oberfläche, beispielsweise am Windelohr, einer Windel zu befestigen, wie bereits vorstehend ausgeführt. Somit ergibt sich ein freies Ende des Bandes, das sogenannte Benutzerende. An diesem ist der Trägerbereich angeordnet, welcher dazu dient, zum Verschließen der Windel nach dem Anlegen vom Benutzer auf eine mit dem Trägerbereich zusammenwirkende Einrichtung am gegenüberliegenden Windelohr geführt zu werden und die Windel somit öffenbar zu verschließen. Der Trägerbereich muss daher betriebssicher die statischen und dynamischen Belastungen abfangen, welche während der Benutzung der Windel auftreten, ohne sich zu öffnen.

Ein Windelverschlussband erstreckt sich vom Befestigungsbereich hin zum den Trägerbereich umfassenden Schließbereich am Benutzerende des Bands in einer Längserstreckungsrichtung. Häufig ist ein Windelverschlussband langgestreckt rechteckig geformt. Auch bei anderen Formen lässt sich eine Längserstreckungsrichtung definieren, wenn diese senkrecht zu einer Längserstreckungsrichtung der Windel bzw. parallel zur Erstreckung eines Benutzerendes des Windelverschlussbandes von der Windel weg angesetzt wird.

Der Trägerbereich definiert sich durch den Umfang um diejenige Fläche des Windelverschlussbandes, die zum Verschlossenhalten der Windel bei anschließender Öffenbarkeit nach dem Gebrauch eingerichtet ist. Innerhalb dieses Bereichs können einzelne Flecken von Zwischenträgern mit darauf angeordneten Komponenten des Verschlusssystems vorgesehen sein. So zeigt beispielsweise die US 2003/0009144 A1 ein solches Windelverschlussband. Die Zwischenträger sind kreisrunde "Inseln", die innerhalb des Trägerbereichs regelmäßig angeordnet oder wahllos platziert sind. Das dortige Band ist im Wesentlichen rechteckig, sodass sich als Längserstreckungsrichtung eine Parallele zur Längskante des Bandes ergibt. Der Rand der Zwischenträger ist vollständig schräg bezüglicher dieser Längserstreckungsrichtung, da er kreisrund geformt ist. Zwar ergeben sich beim Umlaufen jedes einzelnen kreisrunden Zwischenträgers jeweils zwei parallele und normale Tangenten zur Längserstreckungsrichtung; ein rein tangentialer Punkt wird jedoch nicht unter einem "Teilbereich" verstanden, da er keine Erstreckung entlang der Kante hat.

Nach hier an dieser Stelle erläuterten Aspekt der hier vorliegenden Erfindung ist darüber hinaus ein Randflecken mit einem zugehörigen Rand-Zwischenträger vorgesehen. Dieser bildet entlang eines Abschnitts seiner Kontur eine Begrenzung des Trägerbereichs.

Durch die Definition des Trägerbereichs als Fläche innerhalb der Umgrenzung der Flecken liegt die Umgrenzung zwar in der US 2003/0009144 A1 ebenfalls unmittelbar an den dortigen Inseln an, sodass diese entlang eines gerade verlaufenden Umfangs des Trägerbereichs an diesem auch punktuell anliegen. Wie bereits erläutert wurde, ist ein rein punktuelles, tangentiales Anliegen des Fleckens an der Begrenzung des Trägerbereichs jedoch nicht gemeint. Vielmehr soll die Begrenzung des Trägerbereichs entlang eines Abschnitts der Fleckenkante verlaufen. Dabei sei der Trägerbereich immer polygonal umgrenzt - er verläuft also an einer Ecke nicht mit einem Kreisbogenabschnitt eines Fleckens, sondern liegt an einem kreisförmigen an einer Ecke liegenden Flecken genau zweimal tangential an, wobei er zwischen diesen Punkten eine Ecke formt. Bevorzugt kann der Trägerbereich rechteckig geformt sein.

Gegenüber dem Stand der Technik zeichnet sich das hier vorgeschlagene Windelverschlussband durch eine besonders große Scher- und Abhebbelastbarkeit aus. Dies wird insbesondere dadurch erreicht, dass ein breiterer Abschnitt eines Fleckens bis unmittelbar an die Grenze des Trägerbereichs heran reicht. Auf diese Weise lassen sich große Kräfte auch noch an der Grenze des Trägerbereichs mobilisieren. Ein schleichendes Abschälen der beiden Komponenten von einander wird hierdurch sicher vermieden.

Darüber hinaus lässt sich ein dermaßen gestaltetes Windelverschlussband besonders einfach dadurch herstellen, dass in Maschinenrichtung zahlreiche Flecken hergestellt und anschließend beim Trennen der einzelnen Bänder zumindest einige dieser Flecken durchtrennt werden. In diesem Fall kann ein Randflecken vorteilhaft so angeordnet sein, dass derjenige Randabschnitt, der die Begrenzung des Trägerbereichs bildet, auch gleichzeitig über der Kante des Windelverschlussbands liegt.

Es sei darauf hingewiesen, dass ein und derselbe Flecken gleichzeitig Zwischenträger und Randträger sein kann. Es ist jedoch bevorzugt, wenn zusätzlich zu einem oder mehreren zentralen Flecken ein oder mehrere Randflecken vorgesehen sind, welche jeweils von einander getrennte Träger aufweisen.

Wenn als mechanisches Verschlusssystem ein Haken-Schlaufen-Verschluss gewählt ist, liegen die Haken oder die Schlaufen der Komponente im Trägerbereich üblicherweise in Form eines regelmäßigen Gitternetzes angeordnet. Auf Grund der einfachen Herstellungsweise ist dieses Gitternetz üblicherweise mit seinen Achsen parallel beziehungsweise senkrecht zur Längserstreckungsrichtung ausgerichtet. Dies ist auch in der US 2003/0009144 A1 so ausgeführt, der Rand der dortigen Inseln weicht also durch seine kreisrunde Form von der Gitternetz-Regelmäßigkeit mit den beiden Hauptrichtungen vollständig ab.

Kumulativ bzw. alternativ wird, in Abweichung von der US 2003/0009144 A1 vorgeschlagen, dass nicht nur von der Regelmäßigkeit abweichende Fleckenränder, sondern auch ein mit der Regelmäßigkeit verlaufender Randabschnitt eines Fleckens vorgesehen ist. Mit Blick auf die einfache Herstellbarkeit von Gitternetzstrukturen innerhalb der mechanischen Komponenten wird vorgeschlagen, dass der regelmäßige Randabschnitt des Fleckens gerade ist. Ein solcher Flecken lässt sich als Randflecken besonders einfach durch das vorstehend kurz angeführte Herstellungsverfahren erzielen. Hierdurch kommen insbesondere Haken in unterschiedlichen Lagen am Randbereich zu liegen, und werden sogar teilweise an- bzw. durchgeschnitten. Dieses bedingt wesentlich höhere Haltekräfte gerade dieser Haken, so dass auch die Haltekräfte der Gesamtanordnung erheblich gegenüber herkömmlichen Anordnungen ansteigen.

Ein besonders großer Widerstand gegen ein Ablösen der beiden Komponenten des mechanischen Verschlusssystems ergibt sich, wenn im Trägerbereich mindestens so viele Randträger wie sonstige Zwischenträger vorgesehen sind.

Um das mechanische Verhalten des Trägerbereichs möglichst isotrop zu gestalten, können die Flecken innerhalb des Trägerbereichs geometrisch ähnlich oder sogar deckungsgleich ausgeführt sein. Dies kann sich zumindest auf die zentralen Flecken, also die Flecken mit Ausnahme der Randflecken, beziehen. Im Rahmen dieser Anmeldung sollen als geometrisch ähnlich oder deckungsgleich aber auch diejenigen Randträger verstanden werden, welche eine Form gemäß eines Teils eines zentralen Flecken haben. Eine solche Form ergibt sich beispielsweise dadurch, dass - ausgehend von der Form eines zentralen Fleckens - von diesem ein Stück weggeschnitten ist. Die zentralen Flecken können beispielsweise kreisförmig sein, während die Randflecken die Kontur eines Kreisbogenstücks und dessen Sehne haben.

Da besonders hohe Kräfte aufgenommen werden können, wenn möglichst lange Randabschnitte der Randflecken an der Umgrenzung des Trägerbereichs liegen, wird im Falle von kreisrunden zentralen Flecken vorgeschlagen, dass zumindest ein Randflecken halbkreisförmig ausgebildet ist und mit seiner durch den Mittelpunkt des Halbkreises verlaufenden Sehne entlang der Umgrenzung des Trägerbereichs liegt.

Eine besonders guten Kompromiss zwischen der Biegbarkeit des Windelverschlussbands im Trägerbereich und großen Haltekräften erreichen Flecken, welche weniger als ein Drittel einer Breite des Trägerbereichs senkrecht zur Längserstreckungsrichtung, dabei aber mehr als ein Fünftel einer Länge des Trägerbereichs parallel zur Haupterstreckungsrichtung ausmachen.

Um einem Ablösen der beiden Komponenten des Verschlusssystems bestmöglich vorzubeugen, kann ein Zwischenraum zwischen den Flecken, wie vorstehend bereits im Detail beschrieben, eine offene Klebeschicht aufweisen. Insbesondere kann der gesamte Zwischenraum zwischen den Flecken eine solche Oberflächenbeschaffenheit haben. Ein sehr handliches Benutzerende ergibt sich am Windelverschlussband, wenn die offene Kleberschicht auf den Trägerbereich, welcher durch die Randflecken begrenzt wird, beschränkt ist.

Unabhängig von einer etwaigen Klebeschicht können die Flecken vorteilhaft eine eigene Haupterstreckungsrichtung haben. Ein Flecken hat eine eigene Haupterstreckungsrichtung, wenn er eine erkennbare oder ausgeprägte zweidimensionale Struktur mit einer zumindest im Wesentlichen eindimensionalen Ausprägung hat. Als Haupterstreckungsrichtung gilt dann diejenige Richtung, welche entlang derjenigen Hauptachse der Fleckenfläche verläuft, um welche die Fleckenfläche das kleinere Flächenträgheitsmoment aufweist. Bei einem dergestalt langgestreckten Flecken kann auf einfache Weise neben ihm und parallel zu ihm eine Knicklinie vorgesehen sein. Als eine Knicklinie gilt im Rahmen dieser Anmeldung eine gerade oder zumindest im Wesentlichen gerade Strecke, welche zwischen zwei bevorzugt weit beabstandeten Umgrenzungspunkten des Trägerbereichs verläuft und dabei keinen Flecken mit einem Zwischenträger schneidet. Die Zwischenträger sind im Allgemeinen relativ harte und somit unbiegsame Bauelemente aus Kunststoff. Wenn das Benutzerende des Windelverschlussbands eine Knicklinie oder mehrere Knicklinien aufweist, erscheint es in der Hand des Benutzers insgesamt als sehr biegsam und kann Verformungen der Gegenkomponente beim Tragen bestmöglich folgen.

Damit ein Windelverschlussband mit einer Knicklinie der beschriebenen Art nur relativ kontrollierbare Bewegungen ausführt und sich bei aller Biegsamkeit dennoch stabil verhält, kann eine Biegelinie oder eine Schar von Biegelinien vorteilhaft etwa senkrecht oder unter einem Winkel von etwa 45° zur Längserstreckungsrichtung liegen. Unabhängig von der genauen Ausrichtung wird vorgeschlagen, dass nur in einer Richtung oder zumindest im Wesentlichen nur in einer Richtung Knicklinien im Trägerbereich liegen, wodurch eine sehr gute Schließkraft und eine ausreichend hohe Biegemöglichkeit geschaffen wird, die, da die Knicklinien schräg verlaufen, zu einer hohen Verschlusskraft gegen Scherkräfte führt, wie sie gerade von einem verschlossenen Windelverschlussband, welches unter Zug beansprucht wird, ausgeübt werden. Andererseits kann auf die Knicklinien bei der Verwendung von Flecken auch zur Gänze verzichtet werden, wodurch die große Überlappung der Flecken, insbesondere in Längserstreckungsrichtung des Windelverschlussbandes, ebenfalls eine hohe Verschlusskraft gegen Scherkräfte bedingt und dennoch aufgrund der Verwendung einzelner Flecken eine hohe Beweglichkeit des Windelverschlussbandes auch im Trägerbereich gewährleistet wird.

Wenn keine Knicklinien im Trägerbereich liegen, ist dieser bei der Verwendung von Flecken dennoch in sich relativ biegsam, während von der Umgrenzung des Trägerbereichs nur wenig Biegsamkeit ausgeht. Dies führt zu einer sehr kontrollierten haptischen Rückkopplung beim Greifen des Windelverschlussbands und kann vom Benutzer daher als sehr angenehm empfunden werden.

Kumulativ bzw. alternativ wird bei einem Windelverschlussband für ein mechanisches, zwei Komponenten umfassendes Verschlusssystem, bei welchem ein Fasteningträger mit einem Befestigungsbereich und einem Trägerbereich eine Komponente des mechanischen Verschlusssystems in seinem Trägerbereich trägt und diese Komponente auf einem als Flecken ausgebildeten Zwischenträger angeordnet ist, welcher seinerseits an dem Fasteningträger befestigt ist und dessen Rand zumindest in einem Teilbereich schräg bezüglich einer Längserstreckungsrichtung des Windelverschlussbands angeordnet ist, wobei der Zwischenträger sowohl in Längsrichtung als auch in Querrichtung geteilt ist, kann der Zwischenträger sowohl in Längsrichtung als auch in Querrichtung so geteilt sein, dass sich keine Knicklinie durch den Trägerbereich ergibt. Auch hierdurch kann die Haltekraft erheblich erhöht werden, da wesentlich mehr Haken bzw. männliche Komponenten intensiv mit den Schlaufen bzw. mit der weiblichen Komponente verhaken können.

Insbesondere wenn eine Knicklinie vorgesehen ist, aber nicht auf diesen Fall eingeschränkt, ist es von Vorteil, wenn sich der Teilbereich des Fleckens mit dem schrägen Rand über eine gesamte Breite des Windelverschlussbands senkrecht zur Längserstreckungsrichtung erstreckt. Auf diese Weise können insbesondere streifenförmige Flecken besonders einfach hergestellt werden, denn in Maschinenrichtung, also normal zur Längserstreckungsrichtung des Bandes, kann einfach ein durchgehender Streifen mit einem schrägen Rand über mehrere Bänder aufgebracht werden, wobei der durchgehende Streifen beim Trennen der einzelnen Bänder ebenfalls durchschnitten wird. Der schräge Rand eines solchen Streifens kann beispielsweise eine Wellenform oder eine Zickzackform haben.

Es sei darauf hingewiesen, dass ein derartiges Windelverschlussband mit einem Flecken mit schrägem Rand, wobei sich dieser schräge Rand des Fleckens über die gesamte Breite des Bandes erstreckt, bevorzugt mit zwei alternierenden Verlaufsrichtungen, auch unabhängig von sämtlichen übrigen Merkmalen der vorliegenden Erfindung vorteilhaft und erfinderisch ist.

Der Trägerbereich des Windelverschlussbands ist besonders stabil, wenn eine Projektion der Flecken auf eine Gerade, welche in der Bandebene sowie normal zur Längserstreckungsrichtung und/oder parallel zur Längserstreckungsrichtung liegt, zahlreiche verschiedene Überlappungen von Fleckenprojektionen ergibt. Insbesondere ist es von Vorteil, wenn die Flecken so angeordnet sind, dass sich in der Projektion sowohl Flecken überschneiden, welche normal zur Projektionsgeraden im Trägerbereich verstreut liegen, als auch solche, welche im wesentlichen parallel zur Projektionsgeraden beabstandet zueinander in dem Trägerbereich angeordnet liegen.

Ein im Gebrauch sehr stabiles Windelverschlussband ergibt sich unabhängig vom Vorgenannten, wenn der Zwischenträger als Flecken ausgebildet ist, der in Maschinenrichtung einen variierenden Rand aufweist, wobei - in Längserstreckungsrichtung gesehen - über die Wirkungsbreite des Trägerbereichs auf jeder in Maschinenrichtung laufenden Höhe wenigstens ein Flecken vorhanden ist. Mit anderen Worten ausgedrückt ist dies der Fall, wenn keine Knicklinie parallel zur Längserstreckungsrichtung durch den Trägerbereich verläuft; plastisch beschrieben gibt es somit in der Blickrichtung entlang der Längserstreckungsrichtung des Bandes keine Lücke zwischen den Flecken.

Besonders einfach kann ein solcher Zustand dadurch erreicht werden, dass sich der variierende Rand bereits eines Fleckens über die gesamte Höhe des Windelverschlussbands in Maschinenrichtung erstreckt.

An einer Umgrenzung des Trägerbereichs kann ein solcher Flecken vorteilhaft einen gerade verlaufenden Randabschnitt aufweisen.

Außerhalb des Trägerbereichs können eine oder mehrere Klebeflächen vorgesehen sein, um beispielsweise ein Release Tape in der Benutzungsbereitschaft zu halten.

Dementsprechend kann auch ein Windelverschlussband für ein mechanisches, zwei Komponenten umfassendes Verschlusssystem der Zwischenträger durch eine Trennung in einer ersten Richtung und durch eine beabstandeten Schwächung in einer zweiten Richtung als streifen-förmiger Flecken ausgebildet sein. Hierunter ist zu verstehen, dass der Zwischenträger entlang der ersten Richtung vollständig getrennt in zwei oder mehrere Streifen vorliegt, wobei die Streifen somit zu der ersten Richtung und zueinander im Wesentlichen parallel liegen. In der zweiten Richtung, also entlang des Verlaufs der Streifen, sind diese mit einer Randvariation, wie beispielsweise zueinander beabstandeten, vorzugsweise regelmäßigen, Schwächungen, versehen, die insbesondere eine Einschnürung des Streifens, also eine Verjüngung in dessen Verlauf, darstellen können. Auf diese Weise ergeben sich ausgeprägte Knicklinien entlang der ersten Richtung, also entlang der Trennungslinien der einzelnen streifenförmigen Flecken. Demgegenüber ergeben sich etwa senkrecht hierzu schwächere, aber dennoch spürbare weitere Knicklinien. Auch hierdurch können, unabhängig von bzw. auch ergänzend zu den übrigen Merkmalen vorliegender Erfindung bei einem Windelverschlussband die Haltekräfte erhöht werden.

Wenn die Randvariation bzw. die Schwächung der streifenförmigen Flecken senkrecht zur Bandebene vorgenommen ist, so ergeben sich die schwachen Knicklinien als Parallelen zur Längserstreckungsrichtung des Bandes. Wenn hingegen die Randvariationen bzw. die Schwächungen stattdessen oder zusätzlich seitlich an den streifenförmigen Flecken innerhalb der Bandebene vorliegen, so ergibt sich eine Schar von schwachen Knickachsen senkrecht zur Ebene des Bandes.

Um Kräfte bestmöglich aufnehmen zu können, kann der Rand eines streifenförmigen Fleckens zumindest in einem Teilbereich schräg bezüglich einer Längserstreckungsrichtung des Windelverschlussbands ausgeformt sein.

Bevorzugt an einer Umgrenzenden des Trägerbereichs kann ein gerade verlaufender Randabschnitt an dem streifenförmigen Flecken vorgesehen sein, um dort in der eingangs beschriebenen Weise eine hohe Kraftaufnahme zu erreichen.

Auch bei solchen streifenförmigen Flecken kann eine offene Kleberschicht zwischen den Flecken vorgesehen sein, um die Haftwirkung gegenüber der anderen Komponente des Befestigungssystems zu verbessern.

Ein besonders homogenes Verhalten des Trägerbereichs wird erreicht, wenn mehrere deckungsgleiche streifenförmige Flecken innerhalb des Trägerbereichs vorgesehen sind. Der Teilbereich mit dem schrägen Rand kann sich abermals über die gesamte Breite des Windelverschlussbands senkrecht zur Längserstreckungsrichtung erstrecken.

Zahlreiche mögliche Ausgestaltungen gemäß den vorangegangenen Erläuterungen lassen sich besonders praktisch dadurch erreichen, dass bei der Herstellung gezielte Schnitte durch Flecken gelegt werden, wenn die einzelnen Windelverschlussbänder in Maschinenrichtung voneinander getrennt werden. Durch einen Schnitt durch einen Flecken ergibt sich automatisch ein Randflecken mit einer entsprechenden Ausdehnung der Befestigungskomponente entlang des Randes des Trägerbereichs. Der Rand des Trägerbereichs ist in diesem Falle deckungsgleich mit dem Längsrand des Windelverschlussbands.

Es sei darauf hingewiesen, dass sich die vorgenannten Vorteile insbesondere dann auswirken, wenn ein erfindungsgemäßes Windelverschlussband an einer Windel angebracht ist.

Alle vorgestellten Aspekte hinsichtlich der Flecken fußen auf dem Grundgedanken, die Flexibilität des Trägerbereichs gezielt zu beeinflussen und/oder die Kraftübertragung zwischen den mechanischen Verschlusskomponenten durch geometrische Gestaltung der Flecken zu beeinflussen. Durch schräge Ränder der Flecken innerhalb der Bandebene scheinen sich die in der Längserstreckungsrichtung des Bands verlaufenden Zugkräfte besser in das Verschlusssystem einleiten zu lassen, sodass der Verschluss im Gebrauch sicherer geschlossen bleibt. Durch die gezielten Teilungen, Schwächungen, Knicklinien und von Zwischenträgern freien Bereiche wird die Flexibilität der entsprechenden Komponente des mechanischen Verschlusssystems erheblich erhöht, sodass sich diese Komponente besser an ihr Gegenstück anpassen und insbesondere besser mit diesem wechselwirken kann. Die Flexibilität wird in diesem Fall maßgebend von der Flexibilität des Fasteningträgers beherrscht, welcher in der Regel wesentlich flexibler als der Zwischenträger ausgestaltet werden kann.

Durch einen in Maschinenrichtung variierenden Rand eines Fleckens wird die Flexibilität des Trägerbereichs ebenfalls erhöht und auf diese Weise gewährleistet, dass die auf dem Flecken beziehungsweise auf mehreren Flecken vorgesehene Komponente möglichst vielseitig wechselwirken kann, wodurch die Gesamtverschlusskraft weiter erhöht wird.

Im Rahmen dieser Anmeldung beschreibt der Begriff "Maschinenrichtung" diejenige Richtung, mit welcher ein Windelverschlussband üblicherweise eine Maschine während der Produktion durchläuft, wobei es in der Regel senkrecht hierzu durchtrennt wird. Der Begriff Längserstreckungsrichtung" beschreibt eine vom Befestigungsbereich zum Trägerbereich laufende Richtung, die normalerweise senkrecht zur Maschinenrichtung verläuft.

Beim Durchtrennen zumindest einiger der Haken beziehungsweise der hervorstehenden Anordnungen bei der Herstellung eines Verschlussbands oder eines die Haken beziehungsweise die Anordnungen tragenden Fleckes werden diese Bauelemente scharfkantige als in ihrem Ursprungszustand. Auf diese Weise werden die Verhakungseigenschaften und somit die Gesamtverschlusskräfte weiter gesteigert.

Unabhängig von sämtlichem Vorgenannten, aber auch in Addition hierzu, ist es von Vorteil, wenn bei einer Windel mit einem Windelverschlussband und einem Haken-Schlaufen-Befestigungssystem, wobei die Haken im Trägerbereich des Windelverschlussbands getragen sind, die Schlaufen unmittelbar durch ein Nonwoven am Backsheet der Windel gebildet sind. Auf diese Weise kann eine kostenintensive separate Schlaufen aufweisende Komponente des Verschlusssystems entfallen.

Es versteht sich, dass sich die Vorteile der genannten Aspekte der Erfindung bezüglich eines Verschlussbandes auch unmittelbar auf ein Bandmaterial mit einer Längserstreckungsrichtung erstrecken, wobei das Bandmaterial durch Trennungen quer zur Längserstreckungsrichtung und ggf. auch im Wesentlichen in Längserstreckungsrichtung zu Einzelnutzen - bevorzugt ohne Verschnitt - in Form von Verschlussbändern der beschriebenen Form aufteilbar ist. Die EinzeInutzen kommen üblicherweise in Form eines zusammenhängenden Bandmaterials vom Hersteller.

Gleiches gilt analog auch für eine Wicklung, insbesondere eine Rolle oder Spule, eines solchen Bandmaterials. Selbstverständlich gilt gleiches auch für eine Windel mit einem solchen Windelverschlussband.

Die Erfindung wird nachstehend anhand mehrerer Ausführungsbeispiele unter Bezugnahme auf die Zeichnung weiter erläutert. In der Zeichnung können funktional gleiche Bauelemente gleiche Bezugsziffern tragen. Es zeigen
- Figur 1: schematisch in einer Seitenansicht ein Windelverschlussband, zur Erläuterung mehrere Aspekte bei Windelschlussbändern,
- Figur 2: das Windelverschlussband aus Figur 1, in einem zweiten Verarbeitungsschritt angesetzt an ein Windelohr,
- Figur 3: das Windelverschlussband aus den Figuren 1 und 2 in einem dritten Verarbeitungsschritt zum Ansetzen an das Windelohr aus Figur 2,
- Figur 4: das Windelverschlussband aus den Figuren 1 bis 3 in einem vierten Verarbeitungsschritt,
- Figur 5: das Windelverschlussband aus den Figuren 1 bis 4 in einer Verschlusskonfiguration, angesetzt am Windelohr aus den Figuren 2 bis 4 und lösbar verbunden mit einer Landezone, sowie
- Figur 6: schematisch eine Seitenansicht eines ersten Windelverschlussbands,
- Figur 7: das Windelverschlussband aus Figur 1 in der Draufsicht,
- Figur 8: schematisch ein zweites Windelverschlussband in einer Seitenansicht,
- Figur 9: das Windelverschlussband aus Figur 3 in der Draufsicht,
- Figur 10: schematisch ein drittes Windelverschlussband in einer Seitenansicht,
- Figur 11: das Windelverschlussband aus Figur 5 in der Draufsicht,
- Figur 12: schematisch ein viertes Windelverschlussband in einer Seitenansicht,
- Figur 13: das Windelverschlussband aus Figur 7 in der Draufsicht,

- Figur 14: schematisch ein fünftes Windelverschlussband in einer Seitenansicht,
- Figur 15: das Windelverschlussband aus Figur 9 in der Draufsicht und
- Figur 16: schematisch ein sechstes Windelverschlussband in einer Draufsicht.

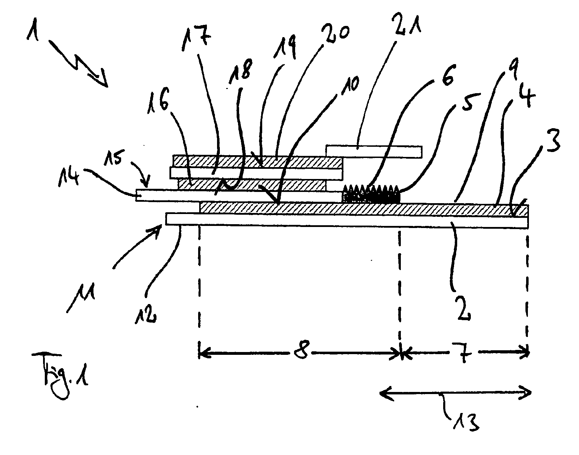

Das Windelverschlussband 1 in Figur 1 besteht im Wesentlichen aus einem bandförmigen Fasteningträger 2, auf dessen Oberfläche 3 eine Klebstofflage 4 vorgesehen ist. Die Klebstoffschicht trägt ihrerseits einen Hakenflecken 5 mit zahlreichen kleinen Haken (durch Zacken dargestellt und exemplarisch mit 6 beziffert).

Der Hakenflecken 5 teilt das Windelverschlussband 1, genauer den Fasteningträger 2, in zwei Bereiche: zum einen in einen Befestigungsbereich 7, zum anderen in einen Schließbereich 8. Der Befestigungsbereich 7 ist dazu bestimmt, permanent an ein Windelohr einer Windel angesetzt zu werden. Beim Ansetzen an das Windelohr wird das Windelverschlussband 1 mit seiner offenen Haftfläche 9 hierzu so an das Backsheet-Material der Windel geführt, dass der Hakenflecken 5 gerade frei bleibt und somit seitlich über das Windelohr der Windel hinweg absteht.

Der Schließbereich 8 dient dazu, die Windel, an deren Windelohr das Windelverschlussband 1 befestigt ist, für eine bestimmte Zeit lösbar verschlossen zu halten. Hierzu gehen die Haken 6 und/oder eine offene Kleberfläche 10 der vom Befestigungsbereich 7 zum Schließbereich 8 durchlaufenden Kleberlage 4 eine lösbare Verbindung mit einer Oberfläche des Hygieneartikels ein. Hierfür ist an der Oberfläche des Hygieneartikels eine Landezone eingerichtet. Üblicherweise werden Windelverschlussbänder der gezeigten Art an beiden Windelohren der beim Tragen rückwärtigen Partie einer Windel angesetzt, dann wird die Windel angelegt und hierbei die Frontpartie der Windel vor dem Bauch gelegt. Auf der Außenseite der Frontpartie ist dann üblicherweise die Landezone vorgesehen.

Beim Entsorgen der Windel wird diese regelmäßig anders gefaltet als während des Tragens. Die Windelverschlussbänder liegen daher üblicherweise beim Verschließen zum Entsorgen an einer anderen Stelle der Windel.

Dadurch, dass der Hakenflecken 5 auf die durchgehende Kleberlage lediglich aufgesetzt ist, ist sichergestellt, dass sowohl die offene Haftfläche 9 des Befestigungsbereichs 7 als auch die offene Kleberfläche 10 im Schließbereich 8 bis unmittelbar an den Hakenflecken 5 heran reichen.

In einer alternativen Ausführungsform kann zwischen Befestigungsbereich 7 und dem Schließbereich 8 noch ein Zwischenbereich vorgesehen sein, der an sich keine unmittelbar verbindende Funktion mit der Windel an sich aufweist, der jedoch den Abstand zwischen Befestigungsbereich 7 und Schließbereich 8 erhöht, so dass der Windelverschluss einfacher zu bedienen ist.

An einem freien Benutzerende 11 des Fasteningträgers 2 hat dieser über den Schließbereich 8 hinausgehend einen Überstandabschnitt 12.

Der Hakenflecken selbst gehört zum Schließbereich 8. Die Haken 6 des Hakenfleckens 5 sind kleine, elastisch biegsame Haken der üblichen Form eines Haken-Schlaufen-Verschlusses, so wie diese beispielsweise in der US 6,210,389 B1 beschrieben sind. Die Haken 6 bilden somit eine männliche Komponente eines mechanischen Zwei-Komponenten-Verschlusssystems, nämlich eines Haken-Schlaufen-Verschlusssystems.

Im Längsverlauf des Windelverschlussbands 1 entlang einer Längserstreckungsrichtung 13 ist der Schließbereich 8 zum freien Benutzerende 11 hin durch die freie Kleberfläche 10 definiert. Zum Befestigungsbereich 7 hin ist der Schließbereich durch den Hakenflecken 5 begrenzt. Die Fläche des offenen Klebers 10 übersteigt die Fläche des Hakenfleckens 5 deutlich. Sie beträgt etwa das Dreieinhalbfache der mit Haken bedeckten Fläche.

Auf der offenen Kleberschicht 10 im Schließbereich 8 ist ein Targetband 14 angeordnet. Das Targetband 14 bedeckt die gesamte offene Kleberschicht 10 und reicht bis unmittelbar an den Hakenflecken 5 heran. Gleichzeitig steht ein Spitzenabschnitt 15 des Targetbands 14 am Benutzerende 11 des Windelverschlussbands 1 über den Fasteningträger 2 hinaus ab.

Auf dem Targetband 14 ist eine zweite Kleberlage 16 vorgesehen, die ein Releasetape 17 trägt. Das Releasetape 17 deckt die gesamte zweite Kleberlage ab und reicht zum Befestigungsbereich hin bis nahe an den Hakenflecken 5 heran, überdeckt diesen jedoch nicht. Zum Benutzerende 11 hin lässt es den Spitzenabschnitt 15 des Targetbands 14 ebenfalls frei.

An einer zur zweiten Kleberlage 16 hin gerichteten Oberfläche 18 ist das Releasetape mit einer Antihaftbeschichtung versehen, sodass der Verbund zwischen der zweiten Kleberlage 16 und dem Releasetape 17 deutlich schwächer ist als der Verbund zwischen der zweiten Kleberlage 16 und dem Targetband 14 sowie deutlich schwächer als der Verbund der offenen Kleberschicht 10 und dem Targetband 14.

An einer Rückseite 19 trägt das Releasetape eine dritte Kleberschicht 20, welche das Releasetape 17 praktisch vollständig bedeckt. An der dritten Kleberschicht 20 ist ein Abdeckstreifen 21 vorgesehen, welcher in einer Projektion auf die Ebene des Fasteningträgers 2 und somit auf die Ebene des gesamten Windelverschlussbands 1 den Hakenflecken 5 vollständig abdeckt.

Das Windelverschlussband 1 kann in der in Figur 1 dargestellten Form als Einzelnutzen aus einem Bandmaterial geschnitten werden.

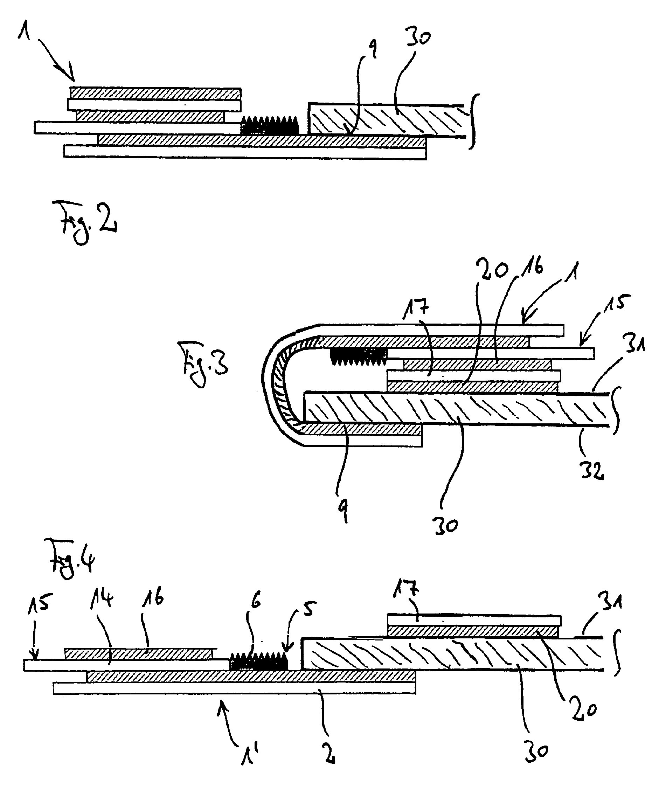

In Figur 2 ist das Windelverschlussband 1 mit der offenen Haftfläche 9 an einer Kante eines Windelohrs 30 (nur teilweise dargestellt) angesetzt. Gleichzeitig ist der Abdeckstreifen 21 entfernt, damit das Ansetzen des Windelverschlussbands 1 und das weitere Handling erleichtert ist.

Im dritten Verarbeitungsschritt ist das Windelverschlussband 1 um die Kante des Windelohrs 30 herum gefaltet (mangels Maßstäblichkeit der Zeichnung bezüglich der Größenverhältnisse verzerrt dargestellt). Die dritte Klebeschicht 20 steht in Eingriff mit einer Innenseite 31 des Backsheets 30. Gleichzeitig ist das Windelverschlussband 1 über die Haftfläche 9 permanent auf der Außenseite 32 des Backsheets 30 fixiert. In dieser Form ist das Windelverschlussband 1 äußerst kompakt am Windelohr 30. Die Windel kann problemlos weiterverarbeitet werden.

Bei einer Erstbenutzung ist das eigentliche Windelverschlussband 1', definiert durch den Fasteningträger 2, vom Releasetape 17 getrennt. Dies kann einfach dadurch geschehen, dass - ausgehend von der Konstellation aus Figur 3 - das Windelverschlussband 1' am Spitzenabschnitt 15 ergriffen und vom Backsheet 30 entfernt wird. Die Verbindung der zweiten Klebelage 16 mit dem Releasetape 17 ist - wie bereits erläutert wurde - relativ schwach. Infolge dessen verbleibt das Releasetape 17 mit der dritten Kleberlage 20 an der Innenseite 31 des Backsheets 30. Der Rest 1' des Windelverschlussbands 1 kann als frei überstehendes Band gegriffen und benutzt werden.

In der Konstellation nach dem Schritt gemäß Figur 4 ist die zweite Kleberlage 16 nunmehr offen. Ebenfalls offen und mit der zweiten Kleberlage 16 relativ nahe benachbart sind die Haken 6 des Hakenfleckens 5.

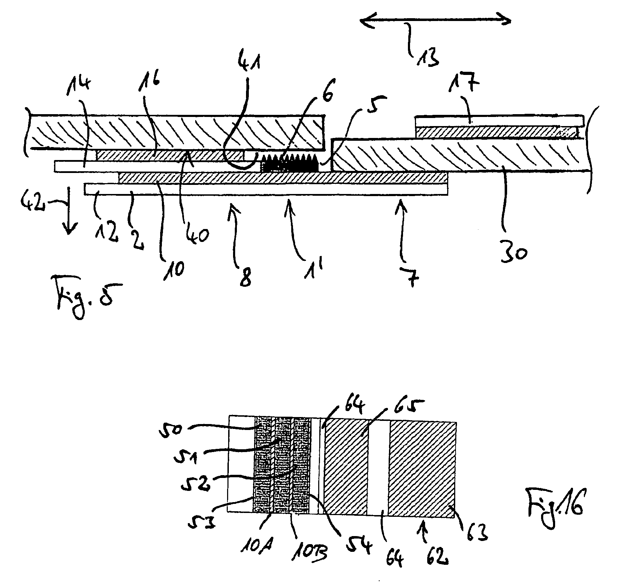

In der offenen Konfiguration nach Figur 4 kann nun der Schließbereich auf eine Landezone am Backsheet aufgebracht werden. Auf diese Weise ergibt sich die Verschlusskonfiguration nach Figur 5.

In der Verbindungskonstellation in Figur 5 ist die Landezone 40 des Backsheets der Windel an die zweite Kleberlage 16 angesetzt, nachdem diese nach dem vierten Schritt offen lag. Bei einem Andrücken des Schließbereichs 8 des Windelverschlussbands 1' gegen die Landezone 40 verbindet sich das Targetband 14 über die zweite Kleberlage 16 mit der Landezone 40 die Haken 6 des Hakenfleckens 5 haken sich in Fasern (nicht dargestellt) eines Non-Wovens als Backsheet der Windel an einer äußeren Oberfläche 41 der Landezone 40 ein. Eine kraftschlüssige Verbindung besteht somit nach ausreichend festem, flächigen Andrücken im Schließbereich 8 gegen die Landezone 40 durch ein Zusammenwirken von Klebekräften 16 und Verhakungskräften 6. Die Haken 6 greifen in die Oberfläche des Nonwoven an der Oberfläche 41 ein.

Das Windelverschlussband 1', genauer dessen Schließbereich 8, ist in der Verschlusskonstellation nach Figur 5 doppelt gesichert: Zum Befestigungsbereich 7 hin schafft zunächst die mechanische Haken-textiles Backsheet-Verbindung eine starke Sicherung gegen ein Verscheren ("shear-off") gegeneinander. Bei einem Verscheren entfernten sich die beiden Teile des Backsheets der Windel senkrecht zur Zeichenebene und/oder entlang der Längserstreckungsrichtung 13.

Die Klebeverbindung des Fasteningträgers 2 über die Klebeschicht 10, das Targetband 14 und die zweite Kleberlage 16 zur Oberfläche 41 in der Landezone 40 sichert insbesondere ab, dass der Schließbereich 8 nicht in eine Richtung 42 von der Landezone abspringen ("pop-off"), sich also senkrecht zur Fläche 41 lösen, kann. Gleichzeitig bewirkt diese pop-off-Sicherung, dass die Haken 6 in der Oberfläche 41 der Landezone 40 gehalten werden, so dass die Verschersicherung zuverlässig aktiv bleibt. Die Verscherung ist die hauptsächlich zu erwartende Kraftbelastung für den Verschluss der Windel.

Die Klebeverbindung zwischen dem Targetband 14 und der Landezone 40 infolge der zweiten Kleberlage 16 ist stärker als die Klebeverbindung zwischen der Klebeschicht 10 und dem Targetband 14. Dies hat zur Folge, dass der Schließbereich 8 des Windelverschlussbands 1' auch in der einmal befestigten Schließkonstellation 5 einfach vom Targetband 14 abgezogen werden kann. Hierzu kann insbesondere der freie Überstandsabschnitt 12 am Benutzerende 11 des Windelverschlussbands 1' ergriffen und in pop-off-Richtung 42 von der Landezone 40 weggezogen werden. Nach Abziehen der Klebeverbindung werden auch die Haken 6 bei weiter fortgehendem Abziehen entlang der Richtung 42 aus der Landezone 40 herausgezogen, so dass die Windel wieder geöffnet ist.

Der Vorgang ist vollständig reversibel: Um die Windel erneut zu verschließen, wird der Schließbereich 8 des Windelverschlussbands 1' entgegen der pop-off-Richtung 42 wieder an die Landezone 40 herangeführt. Diese ist nunmehr permanent mit dem Targetband 14 versehen. Im Detail wird also einfach die offene Kleberschicht 10 wieder an das Targetband 14 herangeführt und angedrückt, wobei durch konsequentes flächiges Andrücken nicht nur die Klebeverbindung wieder hergestellt wird, sondern auch die Haken 6 wieder in die Oberfläche 41 der Landezone 40 eingedrückt werden. Sodann ist die Verbindung der beiden Teile der Windel wieder sicher hergestellt.

Die doppelt wirkende Verschlusssicherung erreicht Widerstände gegen Öffnungskräfte, die eine hohe Zuverlässigkeit des Windelverschlusses gewährleisten: So werden je nach Gestaltung ohne Weiteres pop-off-Kräfte zwischen 0,5 und 35 N von einem Band aufgenommen, welches 25 mm Bandbreite aufweist. Diese Werte beziehen sich auf Umgebungsparameter von 23 ± 2 °C bei 50 ± 5 % relativer Luftfeuchtigkeit, wobei das Band mit einer Abzugsgeschwindigkeit von 200 mm/min. senkrecht zur Verbindungsebene über die gesamte Hakenfläche abgezogen wird, nachdem die gesamte Fläche mit einer Kraft von 20 N angepresst worden ist.

Bezüglich der dynamischen Scherfestigkeit wurden ohne Weiteres Widerstandskräfte zwischen 0,5 und 40 N bei einem 25 mm breiten Band ermittelt, wobei eine Abzugsgeschwindigkeit des Bands von 300 mm/min. innerhalb der Verbindungsebene angesetzt wurde, nachdem die Verbindung mit einem Anpressdruck von 20 N über die Breite des Bands mit einer Geschwindigkeit von 300 mm/min - einmal vorwärts und einmal rückwärts über die Verbindung - hergestellt wurde.

Es sei darauf hingewiesen, dass die vorgestellten Aspekte der Erfindung voneinander unabhängig sind. Das Windelverschlussband 1 in den Figuren 1 bis 5 verkörpert zwar gleichzeitig mehrere Aspekte der Erfindung. So nimmt der Hakenflecken 5 nur einen kleineren Teil des Schließbereichs 8 ein. Darüber hinaus weist der Schließbereich 8 vom Befestigungsbereich 7 aus zunächst die shear-off-Sicherung in Form des Hakenfleckens 5 auf. Des weiteren ist ein separierbares Targetband 14 am Schließbereich 8 angeordnet, welches diesen teilweise abdeckt, nämlich genau denjenigen Teil, der mit der offenen Kleberschicht 10 versehen ist, wobei es die shear-off-Sicherung in Form des Hakenfleckens 5 vollständig ausspart. Das Band würde seine Stärke des doppelten Funktionsmechanismus' aber auch dann ausspielen, wenn beispielsweise das separierbare Targetband nicht vorgesehen wäre und lediglich der Schließbereich 8 mit seinen Haken 6 und der Klebefläche 10 unmittelbar auf eine textile Oberfläche 41 in der Landezone 40 aufgedrückt würde.

Weiterhin sei darauf hingewiesen, dass der Schließbereich 8 nicht streng in genau eine mechanisch wirkende shear-off-Sicherung und eine über Kleber wirkende pop-off-Sicherung aufgeteilt sein muss. Vielmehr kann auch jede der beiden Flächen aufgeteilt sein. Insbesondere kann sogar ein mehrfacher Wechsel zwischen beschichteter, vollständig unbeschichteter und Hakenzone stattfinden.

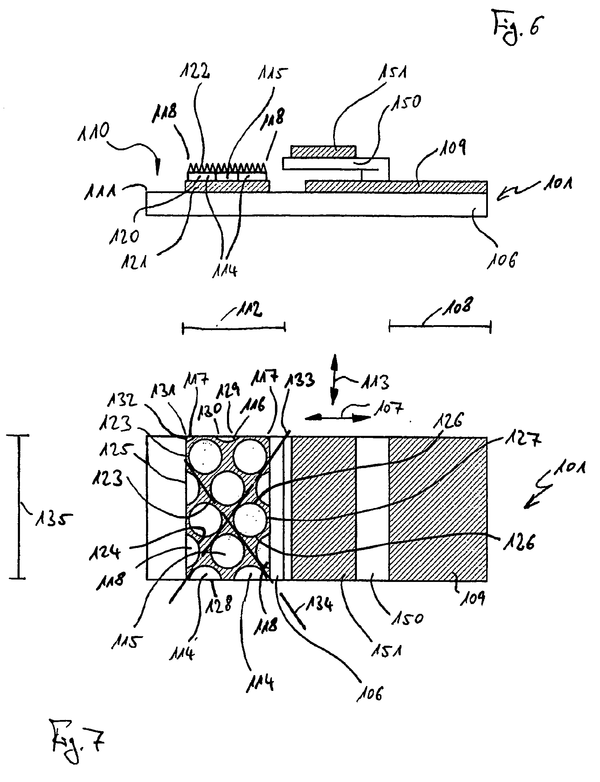

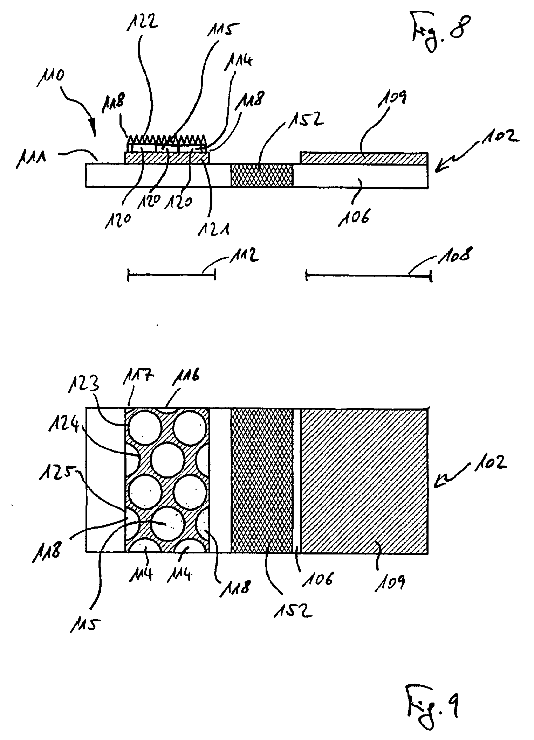

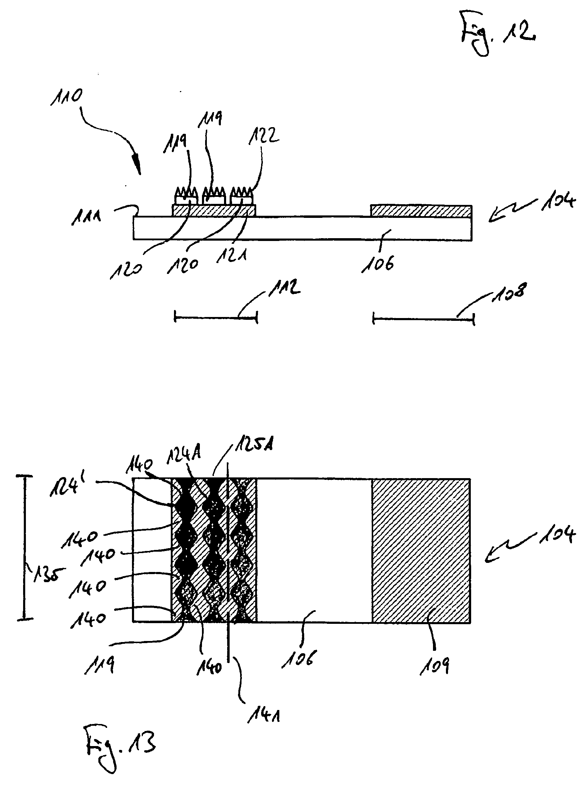

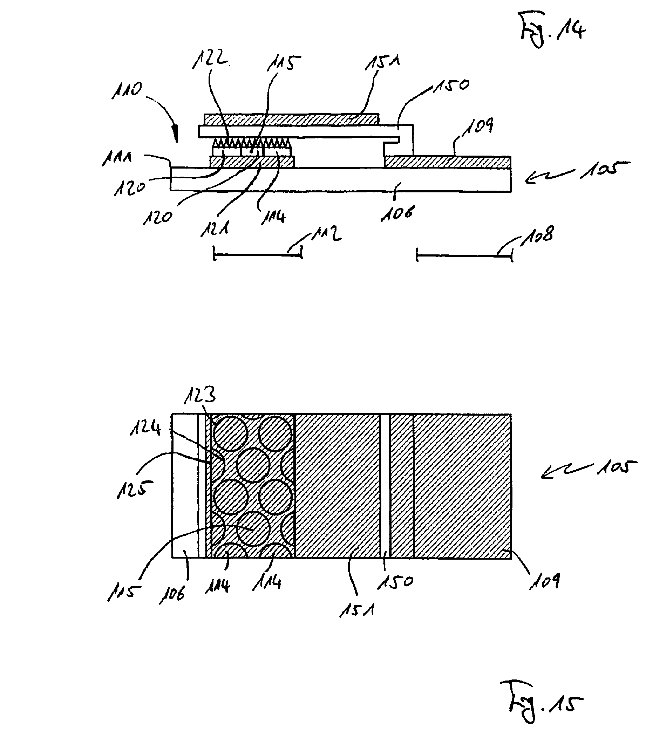

Die Windelverschlussbänder in den Figuren 12, 13 und 16 verkörpern solche Varianten. Die Bänder in den Figuren 12, 13 und 16 tragen im Schließbereich jeweils drei Streifen 119 bzw. 50, 51, 52 mit Hakenmaterial, während sich dazwischen eine offene Kleberschicht 121 bzw. 10A, 10B präsentiert. Beide Bänder haben zudem noch zwei kleine Randstreifen 121 bzw. 53, 54 mit offenem Kleber im Schließbereich. Bei den Windelverschlussbändern in den Figuren 6 bis 11 und 14, 15 finden sich Hakenflächen in Form eines Lochgitters bzw. in Form von Stanzlingen, wobei zwischen den Hakenflächen eine offene Kleberschicht präsentiert wird, wie nachfolgend im Detail erläutert.

Die fünf Windelverschlussbänder 101, 102, 103, 104, 105 nach den Figuren 6 bis 15 bestehen jeweils im Wesentlichen aus einem rechteckigen Fasteningträger 106, auf welchem funktionale Elemente angeordnet sind. Der Fasteningträger 106 ist länglich geformt und hat hierdurch eine Längserstreckungsrichtung 107.

In einem Befestigungsbereich 108 ist auf dem Fasteningträger 106 eine offene Klebeschicht 109, angeordnet. Beim Ansetzen eines der Windelverschlussbänder 101, 102, 103, 104, 105 an eine Windel bei deren Herstellung dient die Klebeschicht 109 des Befestigungsbereichs 108 dazu, das Windelverschlussband 101, 102, 103, 104, 105 permanent an der Oberfläche der Windel, üblicherweise an deren Windelohr, zu fixieren.

Demgegenüber befindet sich an einem freien Ende 110 des Windelverschlussbands 101, 102, 103, 104, 105 derjenige Bereich, welcher beim Anlegen einer Windel vom Benutzer gegriffen werden soll. Dieser Bereich wird auch Fingerlift oder Benutzerende genannt, wobei die Bezeichnung Fingerlift auch oft lediglich einen freien, von Funktionselementen überstehenden Rand 111 des Fasteningträgers 106 bezeichnet.

Am Benutzerende 110 des Windelverschlussbands 101, 102, 103, 104, 105 ist ein Trägerbereich 112 vorgesehen. Der Trägerbereich 112 erstreckt sich bei diesen Ausführungsbeispielen über eine gesamte Breite des Windelverschlussbands 101, 102, 103, 104, 105 in Maschinenrichtung 113. Der Trägerbereich 112 definiert sich durch eine Umgrenzende um Flecken 114, 115, 116, 117, 118, 119 (exemplarisch beziffert) herum, wobei die Flecken 114, 115, 116, 117, 118, 119 jeweils einen Zwischenträger 120 (exemplarisch beziffert) aufweisen, welcher über eine Klebeverbindung 121 an dem Fasteningträger 106 befestigt ist. Auf dem Zwischenträger 120 sind zudem Haken 122 (exemplarisch beziffert) angeordnet, welche eine Komponente eines Haken-Schlaufen-Verschlusssystems bilden.

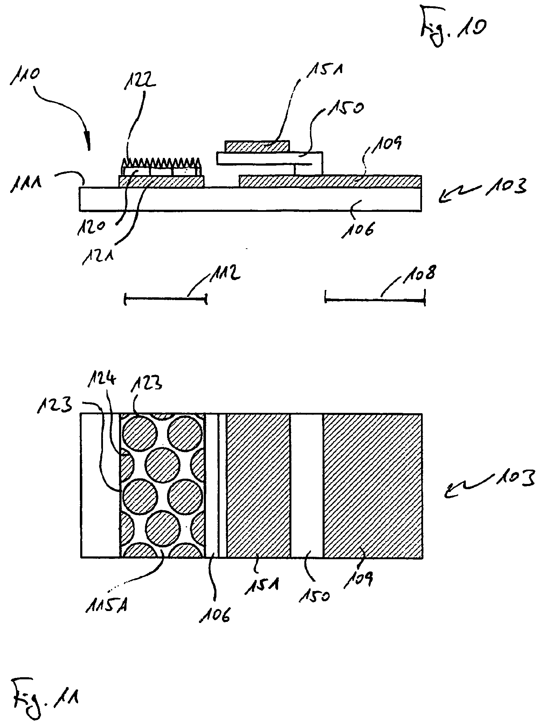

Die Trägerbereiche 112 der Windelverschlussbänder 101, 102 und 105 in den Figuren 6, 7, 8, 9, 14 und 15 haben bezüglich der Fleckengestaltung eine identische Geometrie: so sind sämtliche Flecken 114, 115, 116, 117, 118 Vollkreise oder Kreisausschnitte; während die Trägerbereiche 112 des Windelverschlussbandes 103 in den Figuren 10 und 11 hierzu komplementär sind. Innerhalb des Trägerbereichs 112 liegen bei ersteren Ausführungsbeispielen sechs vollkreisförmige, zentrale Flecken 115. An einem Längsrand des Trägerbereichs 112 und somit senkrecht zur Längserstreckungsrichtung 107 liegen vier halbkreisförmige Flecken 118. An Stirnseiten des Trägerbereichs 112 und somit oberhalb eines Längsrandes des Fasteningträgers 106 liegen zwei ebenfalls halbkreisförmige Randflecken 114 bzw. ein weniger als einen Halbkreis ausmachender Randflecken 116 und zwei weniger als einen Viertelkreis ausmachende Randflecken 117. Die zentralen Flecken 115 sind deckungsgleich zueinander geformt. Die Randflecken 114, 116, 117, 118 sind Ausschnitte von Kreisflächen mit einem untereinander und bezüglich der zentralen Flecken 115 gleichen Radius. Die Kreismittelpunkte der zentralen Flecken 115 und der Randflecken 114, 116, 117, 118 liegen in einem gleichmäßig geformten Raster, sodass Fleckenränder 123, 124, 125 (exemplarisch beziffert) innerhalb des Trägerbereichs 112 ein gleichmäßiges Muster bilden.

Der Fleckenrand 123 der zentralen Flecken 15 beschreibt einen vollständigen Kreis. Insofern ist der Fleckenrand 123 vollständig schräg bezüglich der Längserstreckungsrichtung 107 des Windelverschlussbands 101, 102, 105. Im Detail betrachtet liegt zwar an Quadrantenpunkten 126, 127 (exemplarisch beziffert) eine Tangente parallel (Quadrantenpunkte 126) bzw. senkrecht (Quadrantenpunkte 127) zur Längserstreckungsrichtung 107. Im Detail betrachtet ist dies jedoch nur an den vier Quadrantenpunkten 126, 127 mit einer theoretischen Randstrecke von Null der Fall. Insofern kann nicht davon gesprochen werden, dass an den Quadrantenpunkten 126, 127 ein Teilbereich des Fleckenrandes 123 nicht schräg zur Längserstreckungsrichtung 107 verläuft.

Die halbkreisförmigen Randflecken 114, 118 haben sowohl einen zur Längserstreckungsrichtung 107 schräg verlaufenden Fleckenrand 124 als auch einen geraden Randabschnitt 128 (exemplarisch beziffert). Entlang des äußeren Verlaufs des Halbkreises ist der Fleckenrand 124 somit vollständig schräg zur Längserstreckungsrichtung 107, während der Randabschnitt 128, der mit dem Durchmesser der Randflecken 114, 118 identisch liegt, vollständig gerade und im Falle der Flecken 114 parallel zur Längserstreckungsrichtung 107 bzw. im Falle der Randflecken 118 normal zur Längserstreckungsrichtung 107 verläuft.

Der Randflecken 116 hat ebenfalls einen kreisbogenabschnittförmigen Fleckenrand 124, der jedoch kleiner ist als der derjenige der halbkreisförmigen Randflecken 114, 118. Entlang einer Sehne 129 ist der Randflecken 116 begrenzt. Die Sehne 129 bildet als gerader Fleckenrand 125 des Fleckens 116 einen Randabschnitt 130 in gleicher Weise wie die durchmesserlangen Randabschnitte 128 der halbkreisförmigen Randflecken 114, 118.

Die Eck-Randflecken 117 haben ebenso wie die zentralen Flecken 115 und die übrigen Randflecken 114, 116, 118 einen kreisbogenabschnittförmigen Fleckenrand 124, zusätzlich allerdings zwei senkrecht zueinander stehende, jeweils gerade verlaufende Randabschnitte 131, 132 am Fleckenrand 125. Die Randabschnitte 131, 132 liegen parallel bzw. senkrecht zur Längserstreckungsrichtung 107 des Windelverschlussbands 101, 102, 105, während der kreisbogenabschnittförmige Teilbereich vollständig schräg zur Längserstreckungsrichtung 107 liegt.

Die parallel bzw. senkrecht zur Längserstreckungsrichtung 107 ausgerichteten Randabschnitte 128, 130, 131, 132 der Randflecken 114, 116, 117, 118 liegen an jedem Rand des Trägerbereichs 112 miteinander aufgereiht, sodass sich der Trägerbereich 112 zu einem exakten Rechteck ergibt. Sämtliche geraden Abschnitte der Fleckenränder 123, 124, 125 liegen bei diesen Ausführungsbeispielen auf der Begrenzung des Trägerbereichs 112 und bilden somit entlang der jeweiligen Randabschnitte 128, 130, 131, 132 die Begrenzung des Trägerbereichs mit. Demgegenüber liegen sämtliche schrägen Teilbereiche von Fleckenrändern 124 bzw. sämtliche vollständig schrägen Fleckenränder 123 innerhalb der Umgrenzung des Trägerbereichs.

Die kreisrunden, zentralen Flecken 115 weisen jeweils einen ebenfalls kreisrunden Zwischenträger 120 auf, während die Randflecken 114, 116, 117, 118 einen demgegenüber abgeschnittenen Zwischenträger aufweisen, welcher zumindest im Wesentlichen Form und Größe des jeweiligen Randfleckens hat. Die Klebeschicht 121 verläuft innerhalb des gesamten Trägerbereichs 112 unterhalb der fleckenförmigen Zwischenträger 20 durchgehend. Zwischen den Flecken 114, 115, 116, 117, 118 liegt die Klebeschicht 121 daher offen. Sämtliche Flecken 114, 115, 116, 117, 118 sind mit den Haken 122 in einer sehr feinen, gitternetzartigen Anordnung versehen. Das Gitternetz der Haken 122 ist mit seinen Hauptachsen parallel bzw. senkrecht zur Längserstreckungsrichtung 107 ausgerichtet.

Die gegenüber den kreisrunden Zwischenträgern abgeschnittenen Bauelemente zum Tragen der Haken 122 der Randflecken 114, 116, 117, 118 sind entsprechend der Fleckenform daher Randträger, welche ebenso wie die Flecken bis an den Rand des Trägerbereichs 112 heranreichen.

Die Träger der Randflecken 114, 116, 117, 118 fungieren - im Wortlaut der Patentansprüche - sowohl als Zwischenträger als auch als Randträger, denn sie haben sowohl einen schrägen Teilbereich 124 an ihrem Rand als auch einen Randabschnitt 125, welcher eine Begrenzung des Trägerbereichs 112 bildet.

Die Größe der Flecken 114, 115, 116, 117, 118 ist so gewählt, dass innerhalb des Trägerbereichs und somit auf dem Windelverschlussband senkrecht zur Längserstreckungsrichtung 107 etwa vier vollständige Flecken 115 angeordnet werden können. Gleichzeitig können innerhalb der Trägerbereichs 112 entlang der Längserstreckungsrichtung 107 etwa zwei bis drei vollständige Flecken 115 angeordnet werden. Durch diese Größenwahl ergeben sich relativ stark ausgedehnte Flecken 115, die dem Benutzerende 110 des Windelverschlussbands 101, 102, 105 somit eine relativ große Festigkeit verleihen.

Die Flecken sind gleichzeitig in ihrer regelmäßigen Anordnung so klein ausgeführt bzw. so zueinander angeordnet, dass sich entlang einer ersten Richtung 133 und einer zweiten Richtung 134 jeweils drei gerade Knicklinien ergeben (aus Gründen der Übersichtlichkeit ist nur die längste gebildete Knicklinie gekennzeichnet). Die beiden Richtungen 133, 134 der Knicklinien liegen schräg zur Längserstreckungsrichtung 107. Da keine der beiden Richtungen parallel zur Längserstreckungsrichtung 107 liegt, ergeben sich bei einer Projektion der Flecken 114, 115, 116, 117, 118 auf eine Projektionsgrade 135 innerhalb der Projektion des Trägerbereichs 112 keine freien Strecken. Vielmehr gibt es sogar zahlreiche Überlappungen bei der Projektion der Flecken.

Da die Maschinenrichtung 113 senkrecht zur Längserstreckungsrichtung 107 liegt und die Flecken 114, 115, 116, 117, 118 - und somit auch die Zwischenträger - einen Rand 123, 124 haben, welcher schräg zur Längserstreckungsrichtung 107 liegt und aufgrund der Kreisbogenform variiert, variieren die Fleckenränder - und somit auch die Ränder der Zwischenträger - ebenfalls bezüglich der Maschinenrichtung 113. Dabei ist über die Wirkungsbreite des Trägerbereichs 112, also über die Breite des Bandes 101, 102, 103, 104, 105 auf jeder in Maschinenrichtung laufenden Höhe wenigstens ein Flecken 114, 115,116, 117 und/oder 118 vorhanden. Ansonsten ergäbe sich auch bei Projektion auf die Projektionsflecke 135 eine freie Strecke. Gleichzeitig ergäbe sich eine Knicklinie in eine Richtung parallel zur Längserstreckungsrichtung 107.

Es ist ebenso möglich, die Flecken so zu dimensionieren oder anzuordnen, dass sich keine Knicklinie durch den Trägerbereich 112 ergibt. Hierzu könnten die Flecken beispielsweise teilweise oder sämtlich vergrößert werden. Ebenso wäre es denkbar, das Anordnungsgitter der Flecken in eine Dimension zu strecken. Alternativ oder kumulativ können die Knicklinien dadurch geschlossen werden, dass kleine Flecken in ihren Verlauf gelegt werden oder dass die vorhandenen Flecken zumindest teilweise eine Formänderung erfahren, sodass diese sich durch die bisherigen Knicklinien hindurch erstrecken.

Das Windelverschlussband 103 ist hinsichtlich der Anordnung der Flecken komplementär. Insofern weist dieses Windelverschlussband 103 lediglich einen Flecken 115A auf, der in seinen Rändern jedoch der Geometrie der Windelverschlussbänder 101, 102 und 105 entspricht. Insofern gilt das vorstehend zu der Fleckenanordnung entsprechend, da die vorteilhafte Anordnung der Ränder, Randbereiche, Knicklinien und Schnittkanten auch bei dieser Ausgestaltung verwirklicht sind. Insbesondere die Knicklinien bilden sich durch eine entsprechend ausgerichtete Materialschwächung ebenso, jedoch durch die Löchermittelpunkte laufend aus.

Das vierte Windelverschlussband 104 in den Figuren 12 und 13 weist in seinem Trägerbereich 112 eine andere Geometrie der Flecken 119 auf als die vier übrigen Ausführungsbeispiele 101, 102, 103, 105. Die drei Flecken 119 des Trägerbereichs 112 auf dem Windelverschlussband 104 weisen zwar ebenfalls voneinander getrennte Zwischenträger auf; eine Trennung liegt jedoch nur in Längserstreckungsrichtung 107 des Bandes 104 vor. Entlang der Maschinenrichtung sind die Flecken 119 streifenförmig und erstrecken sich jeweils über die gesamte Breite des Bandes 104. Die streifenförmigen Flecken 119 weichen von einer Rechteckform dadurch erheblich ab, dass sie regelmäßig beabstandete Einschnürungen 140 (exemplarisch beziffert) des Zwischenträgers 120 aufweisen. Der Fleckenrand 124A der streifenförmigen Flecken 119 innerhalb des Trägerbereichs 112 ist über dessen gesamten Verlauf im Wesentlichen zickzackförmig mit ausgerundeten Spitzen geformt. Am Rand des Trägerbereichs 112 weisen die streifenförmigen Flecken 119 jeweils einen geraden Randabschnitt 125A auf, der gleichzeitig über dem Rand des Windelverschlussbands 104 liegt. Die Zwischenträger 120 unter den drei streifenförmigen Flecken 119 sind exakt in der Form der Flecken 119 geformt und vollständig mit Haken 122 besetzt, die in einem sehr engen, mit der bzw. senkrecht zur Längserstreckungsrichtung 107 ausgerichteten Gitternetz angeordnet sind. Der Verlauf der Fleckenränder 124A ist somit nicht nur über den gesamten Randverlauf innerhalb des Trägerbereichs 112 schräg zur Längserstreckungsrichtung 107, sondern gleichzeitig abweichend von der regelmäßigen Anordnung - die ausschließlich parallel zur Längserstreckungsrichtung oder senkrecht hierzu liegt - angeordnet. Da sich die streifenförmigen Flecken 119 bis zum Rand des Trägerbereichs 112 hin erstrecken, fungieren sie gleichzeitig als Randflecken mit einem Randträger, entlang deren Randabschnitten 125A die Begrenzung des Trägerbereichs 112 teilweise gebildet wird.

Zwischen den streifenförmigen Flecken 119 verlaufen in einer ersten Richtung 141 zwei Knicklinien durch den Trägerbereich 112 hindurch. Um diese Knicklinien kann der Trägerbereich besonders einfach aus der Bandebene herausgeknickt werden. Gleichzeitig ergibt sich durch die seitliche Schwächung 140 des Zwischenträgers 120 aus hartem, relativ unbiegsamem Kunststoff die Möglichkeit, eine Teilfläche innerhalb des Trägerbereichs innerhalb der Bandebene um eine Rotationsachse senkrecht zur Bandebene leicht zu verdrehen. Hierdurch wird beim Wirkschluss der Haken 122 mit einer entsprechenden Schlaufenkomponente (nicht dargestellt) einem unbeabsichtigten Lösen der beiden Komponenten durch deren gegenseitiges Verdrehen zueinander sicher vorgebeugt. Selbst wenn bei einer Verdrehung der Komponenten zueinander die Ränder des Trägerbereichs 112 und seines mit Schlaufen versehenen Gegenstücks eine leichte Ablösung erfahren sollten, so kann der mittlere Bereich des Trägerbereichs 112 der Verdrehung leicht folgen und löst sich hierdurch nicht ab.

Die gleiche Wirkung tritt auch bei der Fleckengeometrie nach den Ausführungsbeispielen der Windelverschlussbänder 101, 102, 103 und 105 ein.

Insgesamt zeigen die fünf Ausführungsbeispiele somit Verkörperungen der Erfindung, bei welchen gemäß einem wichtigen Aspekt der Erfindung ein relativ steifer Zwischenträger unter dem mechanischen Verschluss gezielt so getrennt und mitunter zusätzlich geschwächt wird, dass der Trägerbereich einen hervorragenden Kompromiss zwischen Festigkeit und Flexibilität bietet - mit der Möglichkeit, seinem im Verschlusssystem zusammenwirkenden Gegenüber biegsam zu folgen.

Auf den Windelverschlussbändern 101, 102, 103, 104, 105 können zusätzlich Release-Tapes 150 zwischen dem Befestigungsbereich 108 und dem Trägerbereich 112 oder auch über den Trägerbereich 112 hinweg erstreckend vorgesehen sein. Auf den Release-Tapes 150 kann eine Klebeschicht 151 für eine Fixierbarkeit der Release-Tapes 150 vorgesehen sein. Insbesondere können im Verlauf des Fasteningträgers 106 innerhalb oder außerhalb des Trägerbereichs 112 elastische Bereiche 152 angeordnet sein, damit der Trägerbereich 112 Bewegungen seines Gegenstücks noch besser folgen kann.

Es sei nochmals darauf hingewiesen, dass sich durch die vorgeschlagene Verbindungsweise über einen Schließbereich 8 mit einer pop-off-Sicherung und einer shear-off-Sicherung in den beschriebenen erfinderischen Konstellationen ein Windelverschlussband nicht nur sehr zuverlässig haltend herstellen lässt, sondern dieses auch sehr vielseitig einsetzbar ist: So haftet es sowohl gut an einer Außenhaut an dem Backsheet - wie sie beispielsweise bei Inkontinenzwindeln häufig anzutreffen ist - als auch an textilen Oberflächen eines Backsheets - wie sie meist bei Babywindeln zu finden sind.

Auch in der Vielzahl der gezeigten Ausführungsbeispiele und sogar beim Ausführungsbeispiel der Figuren 1 bis 5 zeigt es sich, dass ein Targetband und/oder ein Releasetape vorgesehen sein können, aber nicht notwendigerweise vorgesehen sein müssen, um einen Aspekt der Erfindung zu verwirklichen. Wenn ein Targetband vorgesehen ist, kann dies beim ersten Verschließen der Windel auf dem Backsheet positioniert werden. So kann es eine Landezone für den Klebstoff des Schließbereichs bilden, wenn die Windel geöffnet und wieder verschlossen werden und/oder ein Releasetape auf herkömmliche Weise bei der Herstellung als Niederhalter für den Schließbereich des Windelverschlussbands vor dem Anlegen der Windel dienen soll. Insofern kann auf das Targetband und/oder auf das Releasetape verzichtet werden. Es sei jedoch darauf hingewiesen, dass die explizit vorgeschlagene Konstellation aus einem mechanischen und über einen Kleber schließfähigen Schließbereich sowie ein Targetband im Schließbereich und/oder ein Releasetape im Schließbereich bereits für sich genommen erfinderisch sind.

## Patentansprüche

1. Verschlussband (101, 102, 103, 104, 105) für einen Hygieneartikel, insbesondere für eine Babywindel oder für eine Inkontinenzwindel, mit einem Befestigungsbereich zum permanenten Befestigen am Hygieneartikel und mit einem Schließbereich zum gleichzeitigen lösbaren Verbinden mit einer Oberfläche des Hygieneartikels mit einem mechanischen, zwei Komponenten umfassenden Verschlusssystem, sowie mit fleckenförmigen Trägem (114, 115, 116, 117, 118, 119) innerhalb eines durch sie definierten Trägerbereichs (112), die eine Komponente (122) des mechanischen Verschlusssystems tragen und ihrerseits an einem Fasteningträger (106) befestigt sind, wobei durch den Trägerbereich (112) eine Knicklinie (133, 134) verläuft und wobei der Schließbereich zwei Flächenbereiche aufweist, die Komponenten unterschiedlicher Verschlusssysteme tragen, nämlich der eine Flächenbereich eine shear-off-Sicherung und der andere Flächenbereich eine pop-off-Sicherung, ***gekennzeichnet durch*** kreisrunde zentrale Flecken und zumindest einen halbkreisförmigen Randflecken, der mit seiner **durch** den Mittelpunkt des Halbkreises verlaufenden Sehne entlang der Umgrenzung des Trägerbereichs liegt.

2. Verschlussband nach Anspruch 1, ***dadurch gekenntzeichnet, dass*** die Knicklinie schräg oder normal zur Längserstreckungsrichtung (107) liegt.

3. Verschlussband nach einem der vorhergehenden Ansprüche, ***dadurch gekennzeichnet, dass*** eine Klebeschicht (121) innerhalb des gesamten Trägerbereichs (112) unterhalb der fleckenförmigen Zwischenträger (20) durchgehend verläuft, sodass sie zwischen den Flecken (114, 115, 116, 117, 118) offen liegt.

4. Verschlussband nach einem der vorhergehenden Ansprüche, ***dadurch gekennzeichnet, dass*** durch den Trägerbereich ausschließlich Knicklinien in einer Richtung oder zumindest im Wesentlichen in einer Richtung verlaufen.

5. Verschlussband nach einem der vorhergehenden Ansprüche, ***dadurch gekenntzeichnet, dass*** die Flecken eine eigene Haupterstreckungsrichtung (141) aufweisen.

6. Verschlussband nach einem der vorhergehenden Ansprüche, ***dadurch gekenntzeichnet, dass*** ein Zwischenträger als Flecken ausgebildet ist, der in Maschinenrichtung einen variierenden Rand aufweist, wobei - in Blickrichtung entlang der Längserstreckungsrichtung des Verschlussbandes - über die Wirkungsbreite des Trägerbereichs auf jeder in Maschinenrichtung laufenden Höhe wenigstens ein Flecken vorhanden ist.

7. Verschlussband (104) nach einem der vorhergehenden Ansprüche, ***dadurch gekennzeichnet, dass*** ein Zwischenträger (120) für die Verschlusssystemkomponente (122) durch eine Trennung in einer ersten Richtung und durch eine beabstandete Schwächung in einer zweiten Richtung als streifenförmiger Flecken ausgebildet ist.

8. Verschlussband (104) nach Anspruch 3, ***dadurch gekennzeichnet, dass*** der Zwischenträger entlang der ersten Richtung vollständig getrennt in zwei oder mehreren Streifen vorliegt, wobei die Streifen zu der ersten Richtung und zueinander im Wesentlichen parallel liegen, während n der zweiten Richtung, also entlang des Verlaufs der Streifen, diese mit einer Randvariation, insbesondere mit zueinander beabstandeten, vorzugsweise regelmäßigen, Schwächungen, versehen sind, insbesondere mit einer Einschnürung der Streifen, also eine Verjüngung in deren Verlauf, sodass sich ausgeprägte Knicklinien entlang der ersten Richtung ergeben, also entlang der Trennungslinien der einzelnen streifenförmigen Flecken, und etwa senkrecht hierzu schwächere, aber dennoch spürbare weitere Knicklinien.

9. Bandmaterial mit einer Längserstreckungsrichtung, welches durch Trennen quer zur Längserstreckungsrichtung und gegebenenfalls auch im Wesentlichen in Längserstreckungsrichtung zu Einzelnutzen in Form von Verschlussbändern nach einem der vorstehenden Ansprüche, bevorzugt ohne Verschnitt, aufteilbar ist.

10. Wicklung, insbesondere Rolle oder Spule, eines Bandmaterials nach einem der Anspruch 9.

11. Windel mit einem Verschlussband nach einem der Ansprüche 1 bis 8.

12. Verfahren zum Schließen, Anlegen und/oder zum Entsorgen einer Windel nach Anspruch 11, wobei zum Schließen eine Verbindung zwischen einem Schließbereich eines Windelverschlussbands an einem Windelohr und einer Landezone, bevorzugt an einer Frontpartie, hergestellt wird, ***dadurch gekenntzeichnet, dass*** als Landezone ein Oberflächenbereich eines textilen Backsheets gewählt wird.

## Claims

1. A fastening tape (101, 102, 103, 104, 105) for a hygiene item, in particular for a baby's nappy or an incontinence pad, having a fastening area for permanent attachment to the hygiene item and having a closing area for simultaneous detachable joining to a surface of the hygiene item, having a mechanical fastening system comprising two components, and comprising spot-shaped carriers (114, 115, 116, 117, 118, 119) within a carrier area (112) defined by said carriers, which carry a component (122) of the mechanical fastening system and are in turn fastened to a fastening carrier (106), wherein a bending line (133, 134) runs through the carrier area (112) and wherein the closure area comprises two areas which carry components of different fastening systems, i.e. the one area has a shear-off securing means the other area has a pop-off securing means, **characterised by** circular central spots and at least one semicircular edge spot which lies with its chord running through the mid point of the semi-circle along said boundary of the carrier area.

2. The fastening tape according to claim 1, **characterised in that** the bending lines lies obliquely or normally to the direction of longitudinal extent (107).

3. The fastening tape according to one of the preceding claims, **characterised in that** an adhesive layer (121) runs right through inside the entire carrier area (112) underneath the spot-shaped intermediate carrier (20) so that said layer lies open between the spots (114, 115, 116, 117, 118).

4. The fastening tape according to one of the preceding claims, **characterised in that** bending lines run exclusively through the carrier area in one direction or at least substantially in one direction.

5. The fastening tape according to one of the preceding claims, **characterised in that** the spots have their own principal direction of extent (141).

6. The fastening tape according to one of the preceding claims, **characterised in that** an intermediate carrier is configured as spots having a varying edge in the machine direction whereby, as seen in the direction of the longitudinal extent of the fastening tape, at least one spot is provided at each level running in the machine direction over the effective width of the carrier area.

7. The fastening tape (104) according to one of the preceding claims, **characterised in that** an intermediate carrier (120) for the closure system component (122) is formed by a separation in a first direction and by a spaced-apart weakening in a second direction as strip-shaped spots.

8. The fastening tape (104) according to claim 7, **characterised in that** the intermediate carrier is completely separated into two or more strips along the first direction, wherein the strips lie parallel to the first direction and substantially parallel to one another, whereas in the second direction, i.e. along the profile of the strips, these are provided with an edge variation, in particular with weakenings which are spaced apart from one another, preferably regular, in particular with a constriction of the strips, i.e. a tapering in their profile so that defined bending lines are obtained in the first direction, i.e. along the dividing lines of the individual strip-shaped spots and approximately perpendicular hereto, weaker but nevertheless perceptibly broader further bending lines.

9. A strip material having a longitudinal direction of extent, which can be divided by separating transversely to the longitudinal direction of extent and optionally substantially in the longitudinal direction of extent for single use in the form of fastening tapes according to one of the preceding claims, preferably without cutting.

10. A coil, in particular a roll or spool of a strip material according to claim 9.

11. A nappy having a fastening tape according to one of claims 1 to 8.

12. A method for closing, applying and/or disposing of a nappy according to claim 11, wherein for closing a connection is established between a closing area of a nappy-fastening tape on a nappy wing and a landing zone, preferably on a front section, **characterised in that** a surface area of a textile back sheet is selected as the landing zone.

## Revendications

1. Bande de fermeture (101, 102, 103, 104, 105) pour un article d'hygiène, en particulier pour une couche de bébé ou pour une couche d'incontinence, comprenant une zone de fixation pour la fixation permanente sur l'article d'hygiène et une zone de fermeture pour la liaison amovible simultanée par une surface de l'article d'hygiène avec un système de fermeture mécanique, comprenant deux composants, et avec des supports (114, 115, 116, 117, 118, 119) en forme de pastilles autocollantes à l'intérieur d'une zone support (112) définie par ces supports, qui portent un composant (122) du système de fermeture mécanique et sont fixées pour leur part sur un support d'attache (106), une ligne de pliage (133, 134) passant à travers la zone support (112), la zone de fermeture comprenant deux zones de surface, qui portent des composants de différents systèmes de fermeture, l'une des zones de surface portant une sécurité "shear-off" et l'autre une sécurité "pop-off", **caractérisée par** des pastilles autocollantes centrales circulaires et au moins une pastille autocollante périphérique semi-circulaire, qui se situe avec sa corde passant par le centre du demi-cercle le long d'la délimitation de la zone support.

2. Bande de fermeture selon la revendication 1, **caractérisé en ce que** la ligne de pliage est disposée en biais ou perpendiculairement à la direction d'extension longitudinale (107).

3. Bande de fermeture selon l'une quelconque des revendications précédentes, **caractérisé en ce qu'**une couche adhésive (121) passe de façon continue à l'intérieur de l'ensemble de la zone support (112) au-dessous des supports intermédiaires (20) en forme de pastille autocollante, de sorte qu'elle est disposée ouverte entre les pastilles autocollantes (114, 115, 116, 117, 118).

4. Bande de fermeture selon l'une quelconque des revendications précédentes, **caractérisé en ce que** seules des lignes de pliage passent à travers la zone support dans une direction ou tout du moins principalement dans une direction.

5. Bande de fermeture selon l'une quelconque des revendications précédentes, **caractérisé en ce que** les pastilles autocollantes présentent une direction d'extension principale (141) propre.

6. Bande de fermeture selon l'une quelconque des revendications précédentes, **caractérisé en ce qu'**un support intermédiaire est réalisé sous forme de pastille autocollante, qui présente un bord variable dans le sens de la machine, au moins une pastille auto-collante étant présente, vue le long de la direction d'extension longitudinale de la bande de fermeture, sur la largeur d'action de la zone support sur chaque hauteur passant dans le sens de la machine.

7. Bande de fermeture (104) selon l'une quelconque des revendications précédentes, **caractérisée en ce qu'**un support intermédiaire (120) pour le composant du système de fermeture (122) est réalisé sous forme de pastille autocollante en forme de bande par une séparation dans une première direction et par un affaiblissement espacé dans une seconde direction.

8. Bande de fermeture (104) selon la revendication 7, **caractérisée en ce que** le support intermédiaire est présent le long de la première direction complètement séparée en deux ou plusieurs bandes, les bandes étant sensiblement parallèles à la première direction et parallèles entre elles, alors que dans la seconde direction, donc le long du tracé des bandes, celles-ci sont dotées d'une variation périphérique, en particulier des affaiblissements espacés les uns des autres, de préférence réguliers, en particulier avec un rétrécissement des bandes, donc un rétrécissement dans leur tracé, de sorte que des lignes de pliage prononcées sont obtenues le long de la première direction, donc le long des lignes de séparation des pastilles autocollantes individuelles en forme de bande, et à peu près perpendiculairement à celles-ci d'autres lignes de pliage plus faibles, mais cependant perceptibles.

9. Matériau de bande avec une direction d'extension longitudinale, qui peut être divisé par séparation transversalement à la direction d'extension longitudinale et éventuellement également sensiblement dans la direction d'extension longitudinale par rapport à des rainures individuelles sous forme de bande de fermeture selon l'une quelconque des revendications précédentes, de préférence sans découpures.

10. Enroulement, en particulier rouleau ou bobine, d'un matériau en bande selon la revendication 9.

11. Couche comprenant une bande de fermeture selon l'une quelconque des revendications 1 à 8.

12. Procédé pour la fermeture, la mise en place et/ou pour l'enlèvement d'une couche selon la revendication 11, une liaison entre une zone de fermeture d'une bande de fermeture de couche étant établie pour la fermeture sur un coin de couche et une zone finale, de préférence sur les parties avant, **caractérisé en ce qu'**une zone de surface d'une face arrière textile est choisie comme zone finale.
